# EUROPEAN PATENT APPLICATION

(11) **EP 4 686 507 A1**
(43) Date of publication of application: **04.02.2026**
(21) Application number: 25192555.8
(22) Date of filing: 29.07.2025
(51) Int. Cl.: B01L 3/00, G01N 27/447, G01N 33/543

(54) **SENSOR, DEVICE, SYSTEM AND METHOD**

(30) Priority: 29.07.2024 US 202418787985
(71) Applicant: Analog Devices International Unlimited Company, Limerick, V94 N4V2 (IE)
(72) Inventor: BERNEY, Helen, Limerick, V94 RT99 (IE); PONOMAREV, Youri, Limerick, V94 RT99 (IE); ANTOINE, Christophe, Wilmington, 01887 (US); WU, Joyce, Wilmington, 01887 (US); XIA, Junfei, Wilmington, 01887 (US); DOYLE, Richard, Limerick, V94 RT99 (IE)
(74) Representative: Elkington and Fife LLP

(57) **Abstract**

The present disclosure provides relates to sensors for determining a property of a target analyte in a sample, devices for manipulating a charged species in a sample and systems and methods for determining a property of a target analyte in a sample. These comprise an electric field generator comprising a first electrode element; a second electrode element spaced apart from the first electrode element to define a region therebetween; and a plurality of structures provided within the region, wherein the each of the plurality of structures is configured to concentrate an applied electric field to form corresponding high electric field regions within the region.

## Description

### FIELD OF THE DISCLOSURE

This disclosure relates to sensors, such as biological or chemical sensors, for determining a property of a target analyte in a sample, devices for manipulating a charged species in a sample and systems and methods for determining a property of a target analyte in a sample.

### BACKGROUND

Various biological and chemical assay are known for sensing analytes. Analytes may, for example, include biomarkers, such as hormones, established to assist in patient monitoring and/or diagnosis.

### SUMMARY OF THE DISCLOSURE

The present disclosure provides relates to sensors for determining a property of a target analyte in a sample, devices for manipulating a charged species in a sample and systems and methods for determining a property of a target analyte in a sample. These comprise an electric field generator comprising a second electrode element spaced apart from the first electrode element to define a region therebetween; and a plurality of structures provided within the region, wherein the each of the plurality of structures is configured to concentrate an applied electric field to form corresponding high electric field regions within the region.

In a first aspect, there is provided a sensor for determining a property of a sample, the sensor comprising:
an electric field generator for forming an electric field, comprising:
   a first electrode element,
   a second electrode element spaced apart from the first electrode element to define a region therebetween, wherein the electric field generator is configured to generate an electric field between the first electrode element and second electrode element in the region; and
a sensing assembly configured to provide a measurement signal indicative of a property of a sample provided within or adjacent the region,
wherein the electric field generator further comprises a plurality of structures provided within the region and a first surface comprising at least one structure of the plurality structures; and
wherein each of the plurality of structures is configured to concentrate an electric field applied to the region by the electric field generator to form corresponding high electric field regions such that charged species in a sample provided to the region can interact with the high electric field regions.

In a second aspect, there is provided a method of determining a property of a sample, the method comprising:
providing a sensor comprising:
   an electric field generator for forming an electric field, comprising:
      a first electrode element,
         a second electrode element spaced apart from the first electrode element to define a region therebetween;
   a plurality of structures provided within the region;
   a first surface comprising at least one structure of the plurality structures, wherein each of the plurality of structures is configured to concentrate an applied electric field to form corresponding high electric field regions within the region; and
   a sensing assembly configured to provide a measurement signal indicative of a property of sample within or adjacent the region,
providing a sample to the region,
applying an electric field using the electric field generator to at least one of the plurality of structures to create a high electric field region within the region so as to manipulate charged species within the sample; and
determining a property of the sample based on the measurement signal after manipulation of the charged species.

In a third aspect, there is provided a device for manipulating a charged species in a sample, the device comprising:
an electric field generator for forming an electric field, comprising:
   a first electrode element; and
   a second electrode element spaced apart from the first electrode element to define a region therebetween, wherein the electric field generator is configured to generate an electric field between the first electrode element and second electrode element in the region,
wherein the electric field generator further comprises a plurality of structures provided within the region;
wherein each of the plurality of structures is configured to concentrate an electric field applied to the region by the electric field generator to form corresponding high electric field regions such that charged species in a sample provided to the region can interact with the high electric field regions.
wherein the device comprises a first manipulation region defined by a first structure set comprising at least one of the plurality of structures; and a second manipulation region defined by a second structure set comprising at least one of the plurality of structures; and
wherein the electric field generator is configured to selectively apply an electric field to each of the first manipulation region and the second manipulation region.

In a fourth aspect, there is provided a method of manipulating a charged species in a sample, the method comprising:
providing an electric field generator for forming an electric field, the electric field generator comprising:
   a first electrode element;
   a second electrode element spaced apart from the first electrode element to define a region therebetween, wherein the electric field generator is configured to generate an electric field between the first electrode element and the second electrode element in the region; and
   a plurality of structures provided within the region, wherein each of the plurality of structures is configured to concentrate an applied electric field to form corresponding high electric field regions within the region; and
providing a sample to the region;
selectively applying an electric field to a first structure set comprising at least one structure of the plurality of the structures to create a first manipulation region to thereby manipulate a charged species; and
selectively applying an electric field to a second structure set comprising at least one structure of the plurality of the structures to create a second manipulation region to thereby manipulate a charged species.

In a fifth aspect, there is provided a system for determining a property of a sample, the system comprising:
a sensor according to any of the examples set out herein (for example, the sensor according to the first aspect) and/or the device according to any of the examples set out herein (for example, the device according to the third aspect); and
a controller configured to control the generation of an electric field by the electric field generator.

### BRIEF DESCRIPTION OF THE DRAWINGS

The present disclosure will now be described in more detail with reference to the accompanying drawings, which are not intended to be limiting:
Fig. 1 provides a schematic cross sectional side view of a sensor according to an example;
Fig. 2 provides a schematic cross sectional side view of a sensor according to an example;
Figs. 3A to 3E provide cross-sectional side views of alternative electric field generators which can be used in the sensors and devices according to examples;
Figs. 4A and 4B schematic plan views of parts of electric field generators which can be used in the sensors and devices according to examples;
Figs. 5A to 5C provide cross-sectional side views of parts of electric field generators which can be used in the sensors and devices according to examples;
Fig. 6 provides a schematic plan view of a sensing assembly;
Fig. 7 provides a schematic cross-sectional view of a part of a sensor comprising the sensing assembly of Fig. 6;
Fig. 8 provides a schematic block diagram of a method;
Fig. 9 provides a schematic block diagram of a method; and
Figs. 10A to 10D depict an exemplary structure with a mushroom-type profile and the electric potential (Figs. 10A and 10C) and electric field norm/current density (Figs. 10B and 10D) of the structure.

### DETAILED DESCRIPTION

Electrochemical sensors, including biosensors and chemisensors, are used for various biological and chemical assay are known for sensing analytes in samples. Analytes may, for example, include biomarkers, such as hormones, established to assist in patient monitoring and/or diagnosis.

In a first aspect, there is provided a sensor for determining a property of a sample, the sensor comprising:
an electric field generator for forming an electric field, comprising:
   a first electrode element,
   a second electrode element spaced apart from the first electrode element to define a region therebetween, wherein the electric field generator is configured to generate an electric field between the first electrode element and second electrode element in the region; and
a sensing assembly configured to provide a measurement signal indicative of a property of a sample provided within or adjacent the region,
wherein the electric field generator further comprises a plurality of structures provided within the region and a first surface comprising at least one structure of the plurality structures; and
wherein each of the plurality of structures is configured to concentrate an electric field applied to the region by the electric field generator to form corresponding high electric field regions such that charged species in a sample provided to the region can interact with the high electric field regions.

In a second aspect, there is provided a method of determining a property of a sample, the method comprising:
providing a sensor comprising:
   an electric field generator for forming an electric field, comprising:
      a first electrode element,
      a second electrode element spaced apart from the first electrode element to define a region therebetween;
   a plurality of structures provided within the region;
   a first surface comprising at least one structure of the plurality structures, wherein each of the plurality of structures is configured to concentrate an applied electric field to form corresponding high electric field regions within the region; and
   a sensing assembly configured to provide a measurement signal indicative of a property of sample within or adjacent the region,
providing a sample to the region,
applying an electric field using the electric field generator to at least one of the plurality of structures to create a high electric field region within the region so as to manipulate charged species within the sample; and
determining a property of the sample based on the measurement signal after manipulation of the charged species.

In a third aspect, there is provided a device for manipulating a charged species in a sample, the device comprising:
an electric field generator for forming an electric field, comprising:
   a first electrode element; and
   a second electrode element spaced apart from the first electrode element to define a region therebetween, wherein the electric field generator is configured to generate an electric field between the first electrode element and second electrode element in the region,
wherein the electric field generator further comprises a plurality of structures provided within the region;
wherein each of the plurality of structures is configured to concentrate an electric field applied to the region by the electric field generator to form corresponding high electric field regions such that charged species in a sample provided to the region can interact with the high electric field regions.
wherein the device comprises a first manipulation region defined by a first structure set comprising at least one of the plurality of structures; and a second manipulation region defined by a second structure set comprising at least one of the plurality of structures; and
wherein the electric field generator is configured to selectively apply an electric field to each of the first manipulation region and the second manipulation region.

In a fourth aspect, there is provided a method of manipulating a charged species in a sample, the method comprising:
providing an electric field generator for forming an electric field, the electric field generator comprising:
   a first electrode element;
   a second electrode element spaced apart from the first electrode element to define a region therebetween, wherein the electric field generator is configured to generate an electric field between the first electrode element and the second electrode element in the region; and
   a plurality of structures provided within the region, wherein each of the plurality of structures is configured to concentrate an applied electric field to form corresponding high electric field regions within the region; and
providing a sample to the region;
selectively applying an electric field to a first structure set comprising at least one structure of the plurality of the structures to create a first manipulation region to thereby manipulate a charged species; and
selectively applying an electric field to a second structure set comprising at least one structure of the plurality of the structures to create a second manipulation region to thereby manipulate a charged species.

In a fifth aspect, there is provided a system for determining a property of a sample, the system comprising:
a sensor according to any of the examples set out herein (for example, the sensor according to the first aspect) and/or the device according to any of the examples set out herein (for example, the device according to the third aspect); and
a controller configured to control the generation of an electric field by the electric field generator.

Examples may provide sensors, devices methods and systems which have improved device and sensor functionality and versatility, including an improved ability to manipulate charged species. In the context of sensors, for example, this can increase accuracy of measurement and improved speed of analysis. More generally, devices can have greater control over charged species.

The electric field generator used in these aspects and examples can be used to manipulate charged species e.g. in a solution. The charged species can be the target analyte (where a sensor) and/or it can be a different species. For example, in examples where a sensor is used or the device is used in a sensor system, a target analyte may be a charged species and the electric field generator can be used to manipulate the target analyte. This can be used, for example, to move the target analyte through the system, hold the target analyte relative to the remainder of the solution or to cause interaction with the target analyte with the surface. Alternatively or additionally, it can be used to manipulate other charged species in the sample so as to move them through the system, hold them relative to a target analyte, etc. In some examples, there may be no "target analyte" and, instead, the device can be used for measurement of other properties or general manipulation of species, such as filtering.

The use of the claimed structures (e.g. microstructures) may improve the versatility of the electric field generator and may enable the use of electric fields with different strength profiles and with different concentrations in different areas of the regions. This can allow for customisation of the field for the particular use case and can generally improve the performance. For example, the electric field can have different high strength regions where the electric field is concentrated to attract/retain charged species in particular regions. The structures can also be used to create a gradient across the first surface, for example to drive charged species along a desired path, to provide a focused high electric field to particular desired region or target, thereby permitting additional manipulation and/or as an array to create a structured field (spikey field or stepped field, for example). These examples have many uses, including probing species, cells, moving or filtering charged species (e.g. using the region as a filter with different parts of the region/channel retaining species with different charge strengths through the use of the structures). The structures can also enable the provision of a high electric field without a high external voltage. For example, electric fields of 10MV/m can be generated with <1V applied, enabling use of these in low power systems.

The sensor and method of the first and second aspects, respectively, generally provide these structures to concentrate an applied electric field to form corresponding high electric field regions within a region of the sensor (e.g. a fluid pathway adjacent or in which the sensor is provided). As set out above, this can be used to control interaction of charged species, such as a target analyte, with the sensor, probe a target analyte and/or can be used to filter particular parts out, so that interaction of a target analyte with the sensing assembly is more accurate (for example, by removing charged species which may bind to the sensing assembly). The device and method of the third and fourth aspects, respectively, may be in systems which do not include a sensor (or any transduction means), but in some examples may be present in a system comprising a sensing assembly (as defined herein) or any transduction means. The device and method in these aspects provide different configurations or arrangements of the structures which can be used to control the charged species in more complex manners than was previously achievable. For example, this can be used to provide differently actuatable parts in the region to thereby influence charged species in different regions or cause movement by selective actuation of the different structure sets.

### Electric field generator

### Plurality of structures

The electric field generator comprises a first electrode element (e.g. a field generation element); a second electrode element (e.g. a second field generation element) spaced apart from the first electrode element to define a region therebetween; and a plurality of structures provided in the region, wherein each of the plurality of structures is configured to concentrate an applied electric field to form corresponding high electric field regions within the region. In the first and second aspects and other examples, these are provided on a first surface within the region. In the third and fourth aspects, at least one of the plurality can be provided on the first surface and, in some examples, at least one of the plurality can be provided on another (e.g. a second) surface. In some aspects or examples, there are plural sets of structures with different configurations.

The structures are individual elements or formations provides within the region. In some examples, these can be formed on (e.g. directly on) at least one of the first electrode element or second electrode element. That is, the first electrode element or the second electrode element may define the first surface. In alternative or additional examples, these can be formed on another element located between the first electrode element and second electrode element, for example a layer located between the first and second electrode elements. The structures generate a high electric field. By this is meant a relatively high electric field region as compared to the other parts of the region exposed to the electric field (e.g. other parts of the electric field). In other words, these structures concentrate the electric field in particular areas generating a region of higher strength electric field (for example, around the structures or between adjacent structures) as compared to other parts of the electric field away from the structures. The structures can concentrate the electric field through the shape of the structure, size of the structure, material used, and relative configuration of the structures, for example.

The structures can have a height of from 10 nm to 1000 µm, such as from 100 nm to 20 µm. In some examples, each structure has a height of from 10 nm to 10 µm, such as 10 nm to 2000 nm. This can be from 10 nm to 1000 nm, from 50 nm to 1000 nm, from 100nm to 2000 nm, for example. The height is as measured from the surface on which they are provided, for example the first surface.

The structures can provide the first surface with a raised or recessed profile. The presence of the raised or raised profile can concentrate the electric field at particular points on the surface. In examples, the structures taper to a narrower tip (or apex), where the tip is the point located furthest from the plane of the surface. This can be raised from the surface (where the structures are raised profiles) or into the material defining the surface (where the structures are recesses or apertures in a surface). Such tapering/narrow portions can provide a further concentration of the electric field. For example, in examples, at least one of the structures has a pyramidal shape (including a truncated pyramidal shape) or a conical shape (including a frustoconical shape). In other or additional examples, at least one of the structures has a mushroom shape, inverse pyramid shape (including an inverse truncated pyramidal shape) or an inverse conical shape (including an inverse frustoconical shape). These structures can be a protrusion extending from the first surface extending into the region or a recesses where the opening of the recess is in the region. The recess may be a recess with a base (e.g. a groove) or may be an aperture (i.e. have a second opening in another surface).

In examples, at least one and, in some examples, each of the plurality of structures comprises or is a protrusion extending from the first surface, each protrusion comprising a base portion connected to the first surface (for example, the first electrode element) and a tip portion connected to the base portion; and wherein the tip portion has a width which is different to the base portion. In preferred examples, the tip portion may be smaller than the base portion (as measured at the tip and the base). Such as at least 10%, at least 25%, at least 50% or at least 100% larger. The narrower tip portion can focus the electric field on the tip. In other examples however, the tip portion may be larger than the base portion as measured at the tip and the base. Such as at least 10%, at least 25%, at least 50% or at least 100% larger. Measurement of the widths of the tip portion and base portion can be, for example, at the terminal end of the structure (i.e. where the base joins the surface and at the terminal end of the tip) or, in some examples, as measured at a distance of 10% from the terminal end of the total height of the protrusion. In other examples, at least one and, in some examples, each of the plurality of structures is a recess in the first surface, each recess comprising a surface portion at the plane of the surface of the first electrode element and a tip portion connected to the surface portion; and wherein the tip portion has a width which is different to the surface portion. In some examples, the tip portion may be smaller than the surface portion (as measured at the tip and the surface). In other examples, the tip portion may be larger than the surface portion as measured at the tip and the surface. Such as at least 10%, at least 25%, at least 50% or at least 100% larger. Measurement of the widths of the tip and surface can be, for example, at the terminal end of the structure (i.e. at the surface and at the terminal end of the tip) or, in some examples, as measured at a distance of 10% from the terminal end of the total height of the recess. The recess may be hollow or may be filled, for example with a material different to the material defining the first surface. In some examples, the structures may combine recesses and protrusions. For example, a protrusion can be provided in a recess (the recess being of a different dimension).

In some examples, a (second) material having a different dielectric constant to that of the (first) material defining the first surface may be used as the structure so as to create concentration of the electric field. Permittivity differences can impact the electric field and be used to focus and/or steer the electric field. For example, in some examples, the structures may include at least one recess which may be filled with a material having a different dielectric constant to that of the material defining the first surface. For example, a layer may define or comprise the first surface and comprise a recess, wherein the layer is formed from a first material with a first dielectric constant, and the recess may be at least partially filled with a second material, wherein the second material has a second dielectric constant. The material used as part of the structure (e.g. the second material) can be a solid or liquid, such as an electrolyte). In an example, the recess can be filled with the second material so as to create a planar first surface but with the structures formed in the surface. In other examples, there may be first and second materials (as set out above) in addition to the material of the first electrode element.

The structures may comprise or be formed from metals, metal oxides, metal nitrides, carbon-based materials, a conductive polymer, doped silicon or polysilicon or combinations thereof, In some examples, these are metals (such as copper, nickel, platinum, silver, gold, ruthenium, palladium, molybdenum), including metal oxides (such as anodized alumina, indium tin oxide (ITO), such as an ITO coating on a substrate, ruthenium oxide), metal nitrides (such as titanium nitride), carbon nanotubes, graphene, doped semiconductors or conductive polymers. The structures may be formed of the same material as the layer or structure defining the first surface, or in other examples may be a different material. Example materials for the second material mentioned above include dielectrics selected from a polymer, a glass, a glass-ceramic, a ceramic, a metal oxide, a metal nitride, a silicon-based material or combinations thereof. For example, a polyimide, silicon dioxide, or silicon nitride layer.

Exemplary materials for the first surface and structures include nanoporous gold, anodized alumina, using an acid etch to form a recess or pit in an electrode (such as a rotating disc recessed electrode). In some examples, these may be combined, for example with a protrusion (such as a mushroom protrusion). In some examples, the structures are nanowires (e.g. with at least one dimension <1-1000nm, such as 1-100nm.

In some examples, the structures may be formed from monolayers (such as self-assembled monolayers (SAMs)) of structures, such as beads, such as metallic or dielectric beads (e.g. gold, polystyrene and/or silica beads). The beads can form into lattices with the electric field concentrated in regions around the beads (such as between the beads). These can be from 10nm to 1000nm in their largest diameter, for example. These can be formed on the surface of one of the first or second electrode elements, for example. In some examples, an adhesive layer may be provided on the beads and/or on the surface such that the beads stick to one another and/or the surface.

In examples comprising at least one monolayer of self-assembling structures, the device/sensor may further comprise a plurality of first sub-elements (forming a first electrode element) on which the at least one monolayer of form, each of the first sub-elements being individually actuatable. For example, this could be a row or array of the plurality of first sub-elements. In this way, each area under the monolayer could be selectively actuated to provide manipulation in a different region. This can be useful where the SAM is not uniform across the surface, whether accidental or intended. This would concentrate field lines efficiently by only activating those sub-elements on which sufficient beads are present. Determining which of the first electrode elements to actuate could be determined using e.g. electrical impedance measurements or magnetic measurements (where the beads are magnetic).

In some examples, the region defined between the first electrode element and second electrode element comprises or is a channel. That is, the region may be a channel for retaining fluid (e.g. liquid) such that fluid can be held and, in some case, moved between the first and second electrode elements. This channel can be defined by the first second electrode elements and may be an open channel where at least one side or top of the channel is open, or a closed channel where the only openings are inlet(s) and outlet(s). For example, these may define opposing sidewalls or opposing top and bottom walls of the channel and fluid can be received therebetween. In other examples, a further structure may define the channel and the plurality of structures may be provided within the channel, for example on the further structure (the further structure defining the first surface). The channel can be configured to provide fluid to a sensing assembly, where present. For example, fluid provided to an inlet of the channel may be provided to the sensing assembly. The sensing assembly may be located within the channel or may be provided at or downstream of an outlet of the channel.

In examples, the first electrode element and the second electrode element define a fluid inlet to the region and an opposing fluid outlet from the region, and the plurality of structures are arranged along the length of the region between the fluid inlet and fluid outlet. For example, the region may be an elongated region between the first and second electrode elements, and the plurality of structures may be provided so as to extend along this elongated region. Where the region comprises or is a channel, the plurality of structures may be arranged along a length of the channel. That is, at least two of the structures are arranged along the length (between an inlet and an outlet) of the channel so as to be at different distances along the channel. These maybe provided over a part of the length or the full length of the region (e.g. a channel). Provision of the structures along the length can allow for manipulation of the charged species along the length of the region/channel. For example, this can be used to drive movement of charged species through the region/channel or provide different regions along the length where charged species can be retained. For example, the structures at different positions along the length could be actuated separately or have different strength fields applied thereto to create profiles and gradients across a surface/along the length.

In a further example, the plurality of structures are varied in configuration along the length of the region so as to alter the strength of an applied electric field in different parts of the region. The configuration may be any factor which impacts the strength of the electric field formed in this region at the structure. For example, this can be the size of the structure(s) (e.g. height, width, total volume, and/or shape (e.g. sharpness)) across the surface/along the length or a variation in the configuration of plural structures, such as number, density, and/or pattern. This provides further customisability in the manipulation which can occur across a surface/along the length. In such examples, there can be application of the same electric field to all of the structures but, due to the different location and configurations, there will be different strengths and arrangements of the electric fields.

In some examples, at least one of the plurality of structures is provided on a first surface and at least one of the plurality of structures is provided on a second surface, wherein the second surface is opposite the first surface. This creates an arrangement in which there are high electric fields on opposing surfaces, providing further versatility. For example, this may provide for different electric fields to be applied to each of the first and second surfaces. This may also be used to maximise influence of the high electric field regions on fluid located within the region. In some examples, the first electrode element may define the first surface and the second electrode element may define the second surface. In some examples where the plurality of structures extend along the length, the plurality of structures provided on the first surface and the plurality of structures provided on the second surface are each arranged along the length of the region. In other examples, this may be only on one of the first surface or second surface. In examples comprises structures provided on first and second surface, the structures can be opposite one another. For example, the structures may be arranged as a first set of teeth on the first surface and an opposing second set of teeth. These can be directly opposite or offset from one another. The size of the teeth may be varied along the length so as to alter the field strength at the structures of the length. This can be across both sets of teeth. For example, both may reduce in size in the same direction to create a gradient in the direction.

In the third and fourth aspects, the device comprises a first manipulation region defined by a first structure set; and a second manipulation region defined by a second structure set, wherein the first structure set and second structure set each comprise at least one of the plurality of structures. The first structure set and second structure set are different. The electric field generator is configured to selectively apply an electric field to each of the first manipulation region and the second manipulation region. In examples of the first and second aspects, the sensor comprises a first manipulation region defined by a first structure set; and a second manipulation region defined by a second structure set, wherein the first structure set and second structure set each comprise at least one of the plurality of structures and wherein the first structure set and second structure set are different. The electric field generator is configured to selectively apply an electric field to each of the first manipulation region and the second manipulation region.

The different manipulation regions provide the device/sensor with different means of manipulating charged species, providing further versatility. By "different", it is meant that each structure set is made up of a different selection of the structures. That is, the sets comprise different structures or different combinations of structures such that there are at least two different arrangements of electric field when the sets of structures are actuated. For example, each set can comprise at least one of the plurality of structures. For example, where there are three structures, the first set may comprise one of the structures and the second comprise the other two. In some cases, there may be overlap of the sets or one set may fully encompass the other, depending on the needs. In some examples, the first and second sets may be provided on different surfaces, in others these may be on the same surface (such as the first surface) and in some cases each set may encompass structures on different surfaces. In some examples, the first and second structure sets may differ in the configuration of the plurality of structures. The configuration may be any factor which impacts the strength of the electric field formed in this region at the structure. For example, this can be the size of the structure(s) (e.g. height, width, total volume, and/or shape (e.g. sharpness)) in each set or a variation in the configuration of plural structures, such as number, density, and/or pattern. Provision of the structures along the length can allow for manipulation of the charged species along the length of the region/channel. For example, this can be used to drive movement of charged species through the region/channel or provide different regions where charged species can be retained, enhancing molecular transport.

In these examples, the electric field generator is configured to selectively apply an electric field to each of the first manipulation region and the second manipulation region. That is, the electric field generator is configured such that it can apply an electric field to the first manipulation region independently of the second manipulation region, apply an electric field to the second manipulation region independently of the first manipulation region. This could be through using separate arrangements (e.g. electrodes, or different traces providing different configurations across electrode(s)) for generating the different electric fields or through use of a moveable arrangement where the location of the electric field is altered.

In some examples, the first and second sets (and, where present, further sets) of structures may be arranged along a length of the region. These may have any of the configurations or arrangements as set out for the structures arranged along the length of the region/channel. For example, the structures of each set can could be actuated separately or have different strength fields applied thereto to create profiles and gradients across a surface/along the length.

In one example, the at least one structure of the first manipulation region is provided on the first surface and the at least one structure of the second manipulation region is provided on the second surface.

In one example, the first manipulation region and the second manipulation region are spaced apart so as to form discrete regions. For example, spaced apart by at least 1 µm.

In any of the configurations of the structures, the devices and sensors can be used to probe interactions between charged analytes of interest and either other species or surfaces. For example, the analyte may bind (or adhere) to a surface or a capture species anchored on a surface, and the structures can be used as a probe to detach the bound analyte from the surface or the capture species. In these examples, the first surface may be moveable relative to the surface on which the analyte is adhered or the surface on which the capture species is anchored. The structures can be used as a probe which holds the bound analyte through the use of the electric field and can be used to detach the bound analyte from the surface or capture species, for example by moving the structures (and first surface) away from the surface or capture species to which the analyte is bound. The force required to move the analyte is proportional to the affinity with the surface or capture species (for example, as represented by the association constant Kₐ or dissociation constant K_{D}). This acts as a type of force microscopy. In another or additional example, a tag or detection species may be provided to an analyte (e.g. when the analyte is bound and movement is restricted). The structures can be used to unbind the tag or detection species from the analyte using an electric field. In some examples, the structures can be used to interrogate biological material, such as cells, patches of cell membrane or tissue sections. For example, the devices/sensors can be used as a patch clamp with the structures used to apply a voltage to the sample. For example, the structures (e.g. protrusions) can be used as an electrode to breach a cell wall and measure a response. In other or additional examples, ions can be delivered to particular targets, such as cells or cell walls, by controlling ion movement using the structures. For example, opening and closing of cell wall channels can be controlled by delivering ions (e.g. Na⁺/K⁺) to create action potentials and stimulus response patterns. The use of the structures enables precise control of low concentrations of the ions, rapid changes in the concentration locally around the target, and precise measurements.

### First electrode element and/or second electrode element

Generation of an electric field can be achieved by applying a voltage across the first electrode element and second electrode element. The first electrode element can accordingly be a first electrode and the second electrode element can accordingly be a second electrode. These are located adjacent one another but spaced apart (e.g. by air or an insulator). This can be spaced apart by an amount of at least 100 nm, at least 500 nm, at least 1000 nm. For example, these may be opposite one another, such as directly opposite (parallel to). The sample (e.g. fluid, such as a solution) can be received between the first and second electrode elements.

The first electrode element and/or the second electrode element can comprise or be formed from metals, metal oxides, metal nitrides, carbon-based materials, a conductive polymer, doped silicon or polysilicon or combinations thereof. In some examples, these may be metals (such as copper, nickel, platinum, silver, gold or ruthenium), including metal oxides (such as anodized alumina, indium tin oxide (ITO), such as an ITO coating on a substrate, ruthenium oxide) or metal nitrides (such as titanium nitride) or conductive polymers.

The first electrode element and/or second electrode element can have any form which can be used to generate an electric field. In some examples, the first electrode elements and/or second electrode element may each comprise or be formed from a layer (e.g. a plate), a wire, or any other field generating structure. For example, in some examples, the first electrode element and the second electrode element are two opposing layers.

Accordingly, the first and second electrode element may be adjacent to (i.e. next to or abutting) each other so that the electric field can influence charged species between the elements. This may be that the electric field overlaps with the region therebetween and, importantly, provides an applied electric field to the structures. Where this is used in the context of a sensor, these may be adjacent (i.e. next to or abutting) the sensing assembly, such as the sensing element. That is, these are arranged relative to the sensing assembly (and particularly the sensing element) so that the electric field can influence charged species on the sensing assembly. This may be that the field overlaps with at least one surface of the sensing element.

The first and second electrode element may each be a single, actuatable element or each may made up of sub-elements. For example, each of the first and/or second electrode elements may be made from a plurality of sub-elements which can each be individually actuated (i.e. a voltage applied thereto) to provide different configurations of electric fields. In such examples, the first electrode element and/or second electrode elements may be a unitary part or may be comprised of a plurality of separate (including discrete and spaced apart) components.

### Electric field

By using an electric field to perform manipulation of charged species where the electric field is shaped or amplified in parts by the structures, drawbacks associated with uniform fields or those fields where there is non-uniform field structure but in regions not positively impacting charged species can be avoided. Instead, the structures can be used in numerous different arrangements to provide systems with the flexibility to interrogate species, manipulate molecules and filter out species from a solution on a molecular level. It will also be appreciated that it will not always be possible to know what species will be present: an electric field can be reconfigured or modified in a straightforward manner to tailor the removal step and the success can be monitored simply by measuring the usual response provided by the sensor assembly.

The structures can be used to shape and create advantageous non-uniform electric fields. For example, provide different configurations or arrangements of the structures which can be used to control the charged species in more complex manners than was previously achievable. For example, this can be used to provide different profiles of electric field gradient. This can also be used to achieve electric field strengths that are otherwise not achievable (e.g. in small sensor systems) or require significant power consumption.

In examples, the voltage used to generate the field may be at least 250mV, at least 500mV, at least 750mV, at least 1V. For example 250 mV to 10 V, such as 1V to 5V. For example, where AC is used, this can be from 250 mV to 25 V, such as from 1V to 25V, such as from 1V to 5V. Where DC is used, this can be from 250 mV to 1.5 V. This can be positive or negative. In other examples, lower strength fields may be used, where the voltage across the first electrode element and second electrode element is selected from -750mV to 750 mV. For example, in certain examples, the voltage can be - 500mV to 500mV, 0 to 500 mV, or -350mV to 350 mv, such as 0 to 350 mV. These voltages can be at least one discrete value within these ranges or may comprise a ramp or step through these ranges.

The result force applied to each species will depend on the net electric charge of the species it is acting on. For example, in some examples, the electric field may require a strength of from 0.5 x 10⁶ V/m to 1 x 10⁸ V/m, such as 1 x 10⁶ V/m to 2 x 10⁷ V/m. This may be present in the localised regions around the structures and need not be throughout the entire applied field. It will be appreciated that in any of the examples mentioned here, although the resultant force of the electric field acting on a particular component (e.g. the analyte or non-analyte species) will depend on a number of factors, including the charge on the component, the magnitude of the force will be determined by the magnitude of the electric field such that a higher V/m value will lead to a greater force acting on the species in the sample.

The electric field(s) applied can be adjusted or modified depending on the force require and the use case. In some examples, parameters of the electric field are varied, such as frequency. In examples, any appropriate AC frequency can be used. For example, the ac signal frequency may be 10kHz.

In some examples, the sensor/device comprises a first manipulation region defined by a first structure set comprising at least one of the plurality of structures; and a second manipulation region defined by a second structure set comprising at least one of the plurality of structures; and wherein applying an electric field comprises: selectively applying an electric field to the first structure set; and selectively applying an electric field to a second structure set. The first structure set and second structure set are different. In some examples, at least one of the plurality of structures is provided on a first surface and at least one of the plurality of structures is provided on a second surface facing the first surface; and applying an electric field comprises: selectively applying an electric field to at least one of the plurality of structures on the first surface; and selectively applying an electric field to at least one of the plurality of structures on the second surface.

In some examples, applying an electric field comprises applying an electric field so as to retain charged species (e.g. analyte) within the high electric field region; and wherein the method further comprises moving sample through the region while retaining the charged species (e.g. analyte) within the high electric field region. This can provide a number of useful functions including filtering of the sample, clean up, concentrating the analyte (for example, where the analyte is near the limit of detection of a sensor). In some examples, applying an electric field to at least one of the first structure set or the second structure set comprises applying an electric field to at least one of the first structure set or the second structure set so as to retain charged species (e.g. analyte) within the a high electric field region; and wherein the method comprises moving sample through the region while retaining the charged species (e.g. analyte) within the high electric field region. For example, a target charged species (e.g. analyte) could be held in a retention region of the device/sensor, or may be caused to move more slowly due the charge/size and is effectively retained.

In some examples, the method comprises moving charged species (e.g. analyte) within the region by selectively applying an electric field to the first structure set and to the second structure set. In other examples, the method comprises moving charged species (e.g. other than analyte) within the region by selectively applying an electric field to the first structure set and to the second structure set. This sequential application can drive charged species between structures creating flow of charged species within the region. This can be in one or plural directions (e.g. back and forth). In some examples, this can be from one part of a channel to another. In some examples, there may be more than two structure sets provided along a length of a region or channel so that sequential application of force to these structure sets can be used to move charged species along the region or channel.

As set out above, the properties of the electric field applied can be varied. In some examples, the properties may be actively varied during a manipulation or measurement process. For example, in some examples of any of the aspects or examples set out herein, the electric field can be varied between e.g. higher and lower strength fields (relative to one another). This can be a switch between the states, or the field strength may be varied (e.g. progressively changed between the states). In additional or alternative examples, there may be variation in the applied strength of the field across the region or channel. For example, to provide a gradient or step change between higher and lower strength fields (e.g. alternative higher and lower strength fields). This can be provided by the structures, the parameters used to generate separate electric fields (e.g. there may be plural fields applied to the device or sensor simultaneously, at different parts of the region/channel), or both. For example, the electric field may vary between a minimum and a peak or maximum strength, the peak strength being a strength. This can be achieved by varying the voltage across the electrodes used to generate the field, for example, or could be achieved by other means such as moving the relative position of the first and second electrode elements. In some examples, this variation of the electric field may be selected so as to cause movement of the species within the sample matrix in a single direction. For example, changing from a first value imparting a first directional movement to a second value imparting the same directional movement could be used (e.g. the application of a first positive voltage across the electrodes generating the field followed by a second positive voltage, or the inverse (negative to negative)).

The sensors, devices, systems and methods disclosed herein can be used to manipulate charged species. As set out above, the electric field can be used to manipulate an intended target, such as an analyte, or it can be used to manipulate other species in the sample. The latter can be detection species, or contaminants or other non-targets species, which can otherwise interfere with the sample measurement, or tags or other species.

For example, in cases where there is a sensing surface or element and a target analyte which interacts with the sensing surface or element to generate a measurement signal, the electric field can be used to manipulate charged species. For example, it can be used apply a force to a sample sufficient to move analyte on the sensing element (in a "manipulation step") to interrogate the relationship. Where the analyte selectively binds to a capture species on the surface, this can cause specifically-bound analyte to move on the sensing surface or element. In further examples, the electric field may apply a force to the sample sufficient to detach specifically-bound analyte on the sensing surface/element from a capture species. These steps are particularly effective in view of the structures, which can be arranged about the sensing surface to create localised high-electric field regions where these interactions can be achieved and the interactions can be probed. Manipulation and detachment steps can be used to obtain further information about the analyte. This can be monitored over time to build up an interaction picture and provide further information on the analyte. For example, the kinetics of movement or the specific interactions could be used as a fingerprint indicative of the presence of the analyte and a quantity of the analyte. For example, the response may vary based on the speed of movement or interaction with the sensing surface based on the size and nature of the analyte.

In some examples, the method is for detecting a property of an analyte in the sample and the method further comprises providing a detection species configured to specifically bind to the analyte to the sample to form a bound analyte; and wherein applying an electric field comprises applying an electric field to form a high electric field region and causing the detection species to debind from the analyte using the high electric field region.

In some examples, the electric field can be used to remove any non-specifically-bound analyte and/or non-analyte species from the sensing surface but retain specifically-bound analyte on the sensing element. The removal step can provide significant sensitivity improvements by reducing the signal-to-noise ratio (SNR) by stripping away at least a portion of the non-specifically-bound species from the surface(s) of the sensing element and thereby reducing the interference from these on the measurement signal. The ultimate measurement will be more accurate and limits of detection are reduced. The use of the structures allows for control over the non-specifically-bound species and enables them to be driven away from the sensing surface.

US patent application nos. 18/312,327, 18/312,075 and 18/312,071, which are each incorporated herein by reference, disclose exemplary manipulation processes including manipulation of target analyte using electric fields (removal, detachment (debinding), binding, etc. which processes can equally be applied using the electric field generators disclosed herein.

Detaching the analyte from the capture species or surface (e.g. in a detachment step) or a tag or detection element from the analyte using the high electric field region about the structures requires a force to be applied which is sufficient to overcome the strength of the interaction between these elements. This force applied will depend on the net electric charge of the species it is acting on, and the force required to detach the relevant species will depend on the interaction between the species, such as strength of the bond. These forces are typically in the range of 10 to 400 pN, such as 30 to 400 pN, such as 30 to 300 pN. Accordingly, in examples, during a detachment step, the force applied to the bound analyte is from of 10 to 400 pN. The force applied during the other steps, such as manipulation or removal steps (which may be applied by means other than electric fields), can accordingly be less than this, such as less than or equal to 50 pN, less than or equal to 40 pN, less than or equal to 30 pN or less than or equal to 10 pN. It will be appreciated that a higher force can be applied during manipulation or detachment steps where the binding between the analyte and other species is higher. For example, where a 140 pN force is required to detach an analyte from a capture species, the force applied during manipulation or removal steps may be less than or equal to 100 pN. These forces can be provided by generating an electric field using the voltages mentioned above around the structure, which will provide an electric field concentrating structure. As noted above, the force applied will depend on the net electric charge of the species it is acting on. This force applied will depend on the net electric charge of the species it is acting on. For example, in some examples, the electric field may require a strength of from 0.5 x 10⁶V/m to 1 x 10⁸ V/m, such as 1 x 10⁶V/m to 2 x 10⁷ V/m. This may be present in the localised regions around the structures and need not be throughout the entire applied field.

In examples, the strength or intensity of the electric field applied to the analyte in the manipulation, detachment or removal (of non-specifically bound species). Therefore, in one example, the strength of the electric field is varied during the step of removing at least a portion of the non-specifically-bound analyte and/or non-analyte components from the sensing element. This will provide a different response than if a single value electric field is applied and, if measurements are recorded, can generate additional information on the types of non-specific binding or the state of the sensor assembly. The strength or intensity can be varied using the position of the structure(s) relative to the analyte or the use of different structures.

The application of the electric field to cause detachment (debinding) and/or encourage binding can be a continuous application of a single force. Alternatively, the field may be varied during each step. Similarly, where there are plural debinding and/or binding steps, each iteration may use the same or a different force. Various combinations can be used to provide different information. For example, each may lead to different response thereby providing different information on the binding. For example, different approaches may lead to different detachments and/or interactions between the sensing layer and the species, including analyte species which are specifically bound and species in the sample which are non-specifically bound.

### Sensing Assembly

In the first and second aspects, and in examples of the other aspects, a sensing assembly is present, the sensing assembly being configured to provide a measurement signal indicative of a property of analyte within or adjacent the region. The sensing assembly can be located within the region (e.g. a channel) or may be fluidly connected thereto.

The sensing assembly can comprise a sensing surface (or, in some examples, a plurality of sensing surfaces) which is an active surface for interacting with a sample and is addressable or interrogatable to obtain an indication of the interaction of the solution (e.g. an analyte in the solution) with the surface. By interrogatable, it is meant that it can be addressed or otherwise actuated so as to provide a measurement, such as a measurement signal, indicative of the interaction (e.g. a property). For example, it may be operable to provide a sample measurement signal indicative of a first property of a sample on the sensing surface. The sensing assembly may comprise or be electrochemical sensors (potentiometric, amperometric, impedimetric), optical sensors (absorption, reflection, fluorescence (intensity and/or lifetime)), and/or acoustic sensors (photoacoustic, ultrasound). The property may be selected from an analyte characteristic, conductivity, temperature, or any other property of a relevant fluid. In embodiments, the analyte characteristic may include \detection of the concentration of the analyte in the sample. The sensing assembly may therefore comprise a sensing layer defining or comprising the sensing surface. This sensing layer can be, for example, an electrode defining the surface, or it could be a sensing layer which is interrogated by electrodes (e.g. a dielectric material, such as in a chemiresistor). In examples where the surface is an electrode or a material, the surface can include more than the "bare" surface of these components and can further incorporate additional layers on the active surface. For example, in examples where the surface comprises or is defined by an electrode or sensing surface, the electrode or sensing surface may be functionalized or have further layers thereon. In other examples, the sensing assembly may comprise a separate sensing element or device configured to interrogate the sensing surface. For example, this may be an optical sensing device which is configured to optically measure properties of the surface to obtain an indication of the interaction of the solution with the sensing surface. surface. For example, this may beabsorption, reflection, fluorescence (intensity and/or lifetime) of components on the surface (e.g. sample, analyte or markers indicative of the presence of analyte).

In some examples, where present, the surface may comprise a functional layer. In some examples, the functional layer comprises or is a capture species configured to specifically bind to an analyte. Thus, the sensing assembly may be configured to determine a property of a sample, such as the property of an analyte in a sample (the solution). Any suitable analyte capture species can be selected according to the target analyte which is intended to be sensed by the sensor assembly. For example, each capture species may comprise an antibody with specificity for a particular antigen. In such an example, the analyte may take the form of the antigen. More generally, each capture species may, in some examples, comprise at least one selected from a protein, a peptide, a carbohydrate, aptamer and a nucleic acid. The protein may, for example, be an enzyme, such as an enzyme having specificity for the analyte. In other non-limiting examples, the protein is an antibody. In the latter case, the analyte may be an antigen which is specifically bound by the antibody. Each capture species may, for instance, comprise or be defined by an antigen. In this case, the analyte may be a species, such as an antibody, which is specifically bound by the antigenic capture species. The antigen may be or comprise, for example, a protein, a peptide, a carbohydrate, such as a polysaccharide or glycan. In an example, each capture species is a capture antibody. In an example, each capture species comprises an aptamer. An aptamer may be defined as an oligonucleotide or peptide configured to bind the analyte. Such an aptamer may, for example, be configured to interact with, for example bind, various analyte types, such as small molecules, for example amino acids or amines, proteins, metal ions, and microorganisms.

The sensing layer may comprise or be formed of metals, metal oxides, metal nitrides, carbon-based materials, a conductive polymer, doped silicon or polysilicon or combinations thereof. For example, this may comprise or be formed of copper, nickel, platinum, silver, silver chloride, titanium nitride, ruthenium, ruthenium oxide, or gold. In some examples, this may comprise or be formed from TiO₂ or indium tin oxide (ITO). Where present, the sensing layer may comprise or be a dielectric layer. In examples, the dielectric layer may comprise or be a polymer layer, a glass layer, a glass-ceramic layer, a ceramic layer, a metal oxide layer, a metal nitride layer, a silicon-based layer or combinations thereof. For example, a polyimide, silicon dioxide, or silicon nitride layer. Other sensing layers may include a substrate with a coating on which the anchor species is immobilized. In other examples, the sensing layer may comprise or be formed of carbon (graphene, graphene oxide, or nanotubes), silicon dioxide, aluminum oxide, silicon and/or an electroactive polymer. Sensing layers can provide immobilization of capture species through both covalent-like interactions (e.g. chemisorption of anchor species onto the surface through chemical bond formation) and non-covalent-like interactions (e.g. physisorption of capture species onto the surface through weaker, often van der Waals, interactions) depending on the identity of the surface and the capture species. Provision of capture species or other functionalization to the sensing layer can be achieved through techniques such as spin-coating, physical vapor deposition or electrophoretic deposition, or immersion of the sensing layer in solution.

In some examples, the sensing surface (and in some examples, sensing layer, or alternatively a sensing wire or other structure) can comprise a plurality of sensing recesses (e.g. through holes) formed therein. In such a structure, the measurement signal is dependent on the location of analyte relative to the plurality of recesses. In one example, a sensing layer thus comprises an upper surface and a lower surface and a through hole, the through hole extending from the upper surface to the lower surface, wherein sensing element is configured to provide the measurement signal, the measurement signal being indicative of the interaction of analyte with the sensing element. In some examples, the structures may be pores, such as open or closed pores such that this can also provide the measurement signal, the measurement signal being indicative of the interaction of analyte with the sensing element (and, in particular, the pores). Where these are open pores, these may form through holes extending through the sensing layer, either individually or as part of a porous network of through holes.

Where the sensing layer comprises through holes and/or pores, it may be formed from any of the materials set out above. This may include porous polymer matrices (such as polyimides, polystyrene, or polycarbonate), porous ceramics (such as alumina or silicon dioxide) and glass-ceramic composites. These may, in some cases, be a polymer gel matrix. In other examples, metals oxides, carbon-based materials, or other semiconductor materials (such as silicon, doped silicon, or polysilicon) can be provided with pores or etched through holes.

In certain examples, the sensing surface (including some examples, where this is a sensing layer, or alternatively a sensing wire or other structure) can be suspended between the first and second electrode elements and comprises a plurality of through holes therethrough such that fluid can reside above and below the sensing surface. In other or additional examples, the through holes or recesses may comprise or define the region, such that the structures are provided within the through holes or recesses or on the surfaces of the through hole or recesses. In certain examples of these, the sensing element may define the first electrode element and/or the second electrode element such that the through holes/recesses define the region. For example, in some examples, the sensing element defines the first surface. That is, the sensing element comprises or is the first surface and therefore comprises the structures. In other or additional examples, the through holes or recesses may comprise additional structures having the form of the plurality of structures but in addition to the plurality of structures on the first surface. These additional structures can increase sensitivity to measurements.

In such examples, the measurement signal can be dependent on the location of analyte relative to the through holes/recesses. Such an arrangement has been found to provide a particularly useful tool for determining a property of an analyte, since the relative position of the analyte within or around the through holes/recesses can have a significant effect on the measurement response. In examples where the structures on the first surface are distinct from the through holes/recesses, the structures can be used to move analytes into and out of the through hole/the recess. This is suited to structures where the field is concentrated at a tip of the structure. Such examples enable precise movement of molecules. For example, these can be used in methods to count molecules over time (measure quantitatively).

Moreover, the through holes/recesses provide concentration of the electric field such that the response in response to the relative position of analyte with the through holes/recesses is amplified. In an example, the measurement signal is indicative of an impedimetric property (e.g. dielectric property (e.g. permittivity), resistance, capacitance, impedance, conductance, or a combination thereof) of the sensing element; and wherein the through holes/recesses are configured so that analyte within the structures modifies the impedimetric property, and therefore the measurement signal. That is, it measures an impedimetric property, which in one example is based on the permittivity of the sensing element.

In some examples, the recesses (e.g. wells) or through holes (e.g. via) have a largest diameter (at their widest point) less than or equal to 2 µm, for example from 50 nm to 1 µm. In some examples, the through holes or recesses have a diameter (at their widest point) of less than or equal to 1000 nm, for example less than or equal to 800 nm, less than or equal to 500 nm. This may be from 1 nm to 2000 nm, 1 nm to 1000nm, 1 nm to 800 nm, 1 nm to 500 nm, such as 10 nm to 1000 nm, 10 nm to 800, 10 nm to 500 nm, 50 nm to 2000 nm, 50 nm to 1000 nm, 50 nm to 800, 50 nm to 500 nm, 100 nm to 1000 nm, 100 nm to 800, or 100 nm to 500 nm. In one example, the recesses or through holes have a narrowest diameter of at least 0.5 nm, for example at least 1 nm, or at least 10 nm. Recess or through hole radius will impact the response to an analyte: a larger recess or through hole will have a response which is more dependent on the materials within it. A smaller recess or through hole may be selective to particular analytes.

In some examples, the electrode can be an interdigitated electrode (IDE) comprising a first electrode and a second electrode, the first and second electrodes arranged in an interdigitated configuration. By interdigitated configuration, it is meant that the first electrode and second electrode are interdigitated with at least one of the electrodes surrounding at least a part of the other. This can include fingers which are interlaced or interlocked and may include interdigitated comb, spiral or serpentine configurations. In such examples, a sensing layer (of the sensing assembly) can be provided between the fingers of the electrode, for example in the manner set out in US patent application nos. 18/312,327, 18/312,075 and 18/312,071, which are incorporated herein by reference. These can include sensing layer(s) with through holes/recesses, as set out herein. The present of the IDE enables the miniaturisation of the system by eliminating the need for a reference electrode. Typical systems require bulky reference electrodes or pseudo reference electrodes which can have performance problems. Together, these enable the provision of a miniaturised yet accurate sensing system. In some examples, the plurality of structures can be provided on the IDE, for example the IDE fingers.

As set out in some examples above, there may be some examples in which the first surface is defined by a part of the sensing assembly, e.g. a sensing layer. This allows for dual purposes of field creation and sensing. For example, the electric field can be modulated and sensed at the same time. As set out above, this can be an indicator of an impedimetric property (e.g. using two electrodes to sense capacitance). For example, there may be structures arranged around flatter portions of an electrode where the sensing can occur (where the electric field is lower). Exemplary structures for this purpose include recesses or through holes in a sensing IDE or sensing rotating disk electrode or protrusions on a sensing IDE or a sensing rotating disk electrode. For example, impedimetric properties (such as impedance or dielectric change) could be examined in the regions between the high fields.

The methods, systems and sensing assemblies disclosed herein can be used to determine or measure a property of a sample, such as the temperature, electrical conductivity, a property of an analyte in the sample. In certain examples, this can be selected from the concentration of the analyte in the sample, the diffusion constant of the analyte (e.g. rate of diffusion measured in m²/s) in the sample matrix, or a combination thereof. The terms "analyte concentration" or "concentration of the analyte" as used herein may, in certain examples, refer to the activity of the analyte. The activity of the analyte may provide a measure of the effective concentration of the analyte in a sample matrix. In one example, the measurement signal is indicative of an impedimetric property of the sensing layer; and wherein the plurality of structures are arranged on or in the sensing layer such that an analyte received between or within the structures modifies the impedimetric property.

Using the electric field provided by the structures enables these properties to be determined more accurately and/or more quickly. The field can be used to manipulate analyte and then this can be monitored over time to build up an interaction picture and provide further information on the analyte. For example, the kinetics of movement or the specific interactions could be used as a fingerprint indicative of the presence of the analyte and a quantity of the analyte. For example, the response may vary based on the speed of movement or interaction with the sensing parts (e.g. through holes/recesses based on the size and nature of the analyte) and even the structures on the first surface.

### Charged Species, Analyte and Capture Species

The electric field generator can be used to manipulate charged species in a sample, for example, in a solution. Charged species can be one species (e.g. one molecule, or one type of species) or plural species. This can be, for example, biological charged species, such as DNA, charged biomolecules, etc, or more generally charged species such as ions. These can be in a solvent or may comprise a liquid, gas, etc. The charged species can be the target analyte (where a sensor) and/or these can be different species. For example, in examples where a sensor is used or the device is used in a sensor system, a target analyte may be a charged species and the electric field generator can be used to manipulate the target analyte. This can be used, for example, to move the target analyte through the system, hold the target analyte in a particular region and/or manipulate the target analyte on the sensing assembly to provide changes in the measurement signal. In other examples, the charged species may be any other species in the sample. For example, it may be used to move charged species through the system, hold charged species in a particular region and/or manipulate the charged species on the sensing assembly to provide changes in the measurement signal.

The analyte may, for example, be selected from a molecular species, a metal ion, a virus, and a microorganism. Biomolecule analytes are particularly useful and may, for instance, be a hormone selected from an eicosanoid, a steroid, an amino acid, amine, peptide or protein, a nucleic acid, single or double stranded DNA, peptide nucleic acid. Sample matrix refers to the sample as a whole, including the analyte if present. It may comprise a carrier (such as a liquid) and the analyte. The sample matrix may be, for example, blood, urine, sweat, tears, etc., and may (potentially) contain the analyte. In an example, the sample matrix is a liquid.

In examples, the sensing surface (e.g. the sensing element defining the surface) may comprise a capture species configured to selectively bind to a target analyte. Any suitable analyte capture species can be selected, according to the analyte which is intended to be sensed by the sensor assembly. For example, the capture species may comprise an antibody with specificity for a particular antigen. In such an example, the analyte may take the form of the antigen. More generally, the capture species may, in some examples, comprise at least one selected from a protein, a peptide, a carbohydrate, a nucleic acid, and an aptamer. The protein may, for example, be an enzyme, such as an enzyme having specificity for the analyte. In other non-limiting examples, the protein is an antibody. In the latter case, the analyte may be an antigen which is specifically bound by the antibody. The capture species may, for instance, comprise or be defined by an antigen. In this case, the analyte may be a species, such as an antibody, which is specifically bound by the antigenic capture species. The antigen may be or comprise, for example, a protein, a peptide, a carbohydrate, such as a polysaccharide or glycan. In an example, the analyte capture species comprises an aptamer. An aptamer may be defined as an oligonucleotide or peptide configured to bind the analyte. Such an aptamer may, for example, be configured to interact with, for example bind, various analyte types, such as small molecules, for example amino acids or amines, proteins, metal ions, and microorganisms. In one example, the capture species comprises at least one aptamer selected from an oligonucleotide, a polynucleotide, a peptide, or a combination thereof. Such aptamers, as well as other biological species such as antibodies, are well suited to the sensor assemblies and methods disclosed herein.

The capture species (for example, a plurality of individual capture species) can be located adjacent to (i.e. next to or abutting) or on the sensing surface. The interaction between the analyte and the capture species will alter the measurement signal obtained. In some examples, the capture species is provided on (e.g. adhered to or bound to) the sensing surface (e.g. the surface of an electrode or sensing layer). In some examples, the sensing surface is functionalized with the capture species. Such functionalization can be achieved in any suitable manner, such as by covalently or non-covalently immobilizing the capture species to the surface. In examples comprising through holes and/or recesses on the sensing surface, the capture species may be provided adjacent or in the through holes and/or recesses.

In some examples, a detection species or tag may be provided to the sample, wherein the detection species is configured to specifically-bind to the analyte (e.g. a part other than that to which the capture species can bind, where a capture species is also present). The detection species may be provided when the analyte is specifically bound to the capture species or before this occurs. The detection species may be an amplification species for amplifying the measurement signal or response, or may be a different species, for example provided with the purpose of increasing the selectivity of the measurement. This can in examples be as part of a sandwich assay. In some examples, this can be used to create a charge on a species by providing a charged species as the tag.

In any of the configurations of the structures, the devices and sensors can be used to probe interactions between charged analytes of interest and either other species or surfaces. For example, the analyte may bind (or adhere) to a surface or a capture species anchored on a surface, and the structures can be used as a probe to detach the bound analyte from the surface or the capture species. In these examples, the first surface may be moveable relative to the surface on which the analyte is adhered or the surface on which the capture species is anchored. The structures can be used as a probe which holds the bound analyte through the use of the electric field and can be used to detach the bound analyte from the surface or capture species, for example by moving the structures (and first surface) away from the surface or capture species to which the analyte is bound. The force required to move the analyte is proportional to the affinity with the surface or capture species (for example, as represented by the association constant Kₐ or dissociation constant K_{D}). This acts as a type of force microscopy. In another or additional example, a tag or detection species may be provided to an analyte (e.g. when the analyte is bound and movement is restricted). The structures can be used to unbind the tag or detection species from the analyte using an electric field.

### Transduction

In the methods and system disclosed herein, the transducer mechanism(s) used to determine the measurement signal may be any suitable method of transduction. For example, this may comprise measuring the potential (e.g. voltage), current, permittivity, charge and/or frequency. In some examples, the sensing element comprises at least one working electrode either forming the sensing surface or adjacent and addressing an element forming the sensing surface. In some examples, changes in or interactions with the functional layer formed by the capture species on the sensing surface can be detected through changes in potential. In other examples, changes may be determined by monitoring changes in current passing through each sensing site (at a constant potential).

The methods and systems can be used to determine or measure a property ("analyte property") of an analyte in a sample, such as an analyte characteristic. In certain examples, this can be selected from a determination of the presence of the analyte, the concentration of the analyte in the sample, the diffusion constant of the analyte (e.g. rate of diffusion measured in m²/s) in the sample matrix, the binding affinity of the analyte to the capture species or a combination thereof. The terms "analyte concentration" or "concentration of the analyte" as used herein may, in certain examples, refer to the activity of the analyte. The activity of the analyte may provide a measure of the effective concentration of the analyte in a sample matrix.

The sensors, systems and methods disclosed herein can comprise determining the property of a sample, such as the property of an analyte, based on the measurement signal. That is, the sensing assembly (e.g. sensing layer) can be interrogated or addressed to provide a measurement signal indicative of the interaction between the sensing surface and the sample. Changes in these interactions will lead to a change in the measurement signal, which can be equated to a property. The method may further comprise obtaining the measurement signal during and/or after manipulation using the electric field. Interrogation using the manipulations set out herein provides additional information over usual sensors and allows for a comparison of binding vs debinding, which can provide further information and discriminate between species and types of binding.

Moreover, these can be used to provide information not obtainable through traditional assays, such as confirmation of binding affinity, for example. This can be useful for examples in which there may be mutations of modifications of the analyte. For example, DNA mismatch is one type of mutation which is very difficult to detect. For example, where DNA is the analyte, it may be that some of the DNA has not replicated correctly or there has been a mutation such that there is DNA mismatch. This mismatch may not be significant enough to prevent binding with the capture species, but it will impact the binding affinity between the DNA and the capture species. Accordingly, the response to the binding and debinding will therefore be different, and signal changes associated with each can provide information on the amount of each present in a sample. In the case of the application of force, it might be that the mismatched DNA species debind at an earlier point that the target DNA, resulting in an earlier change in signal. The rate of binding may also be slower. This could be used as a way to reduce errors of replication during PCR, for example.

In some examples, the determination of the property may be based on a parameter relating to the change in measurement signal, such as rate of change and/or magnitude. The rate of change can provide useful information about the kinetics and nature of the non-specific binding during the process. For example, the information over time and changes in the rate may give information about the types of species bound to the sensor element. For example, at low concentrations, the time required to reach equilibration is longer (equilibration is concentration dependent). For low dissociation rates in standard assays, the longer the incubation time required to reach equilibration. Performing manipulation (such as binding and debinding) and using the signal (1) reduces incubation time by driving association kinetics with locally increased analyte concentration and (2) increases the dissociation rate by driving the debinding, thereby increasing measurement speed. This may give rise to a specific fingerprint or pattern, providing further information on the charged species and in turn how to compensate or obtain an accurate reading. This fingerprint or pattern can be compared to what is expected (e.g. a predetermined fingerprint or pattern) for an analyte to determine the presence and/or concentration of the analyte. Accordingly, in some examples, the property determination unit is configured to determine the property of the analyte based (at least in part) on a rate of change in the measurement signal during at least one of the binding and/or debinding steps. This may be during both and/or all measurement time.

Using an electric field with high electric field regions provides a quick, effective and selective means of causing manipulation of charged species. For example, fields can be reconfigured or modified in a straightforward manner to tailor the influence. This provides versatility that different chemical-based solutions could not. Moreover, the generation of a field can be carried out using electrodes and/or magnetic elements which can readily be integrated into existing sensor structures and semiconductor manufacturing techniques.

### System

In an aspect, a system for determining a property of a sample comprises a sensor according to any of examples disclosed herein and/or a device according to any of the examples disclosed in; and a controller configured to control the generation of an electric field by the electric field generator. The system may be configured to perform any of the method steps disclosed herein. For example, the controller may be configured to operate the sensing assembly to perform any of the method steps herein. Moreover, any of the examples set out herein with respect to the method apply equally to the system, and any of the examples set out herein with respect to the system apply equally to the method.

In one example, the system is for determining the property of an analyte, the system further comprises a detection species configured to specifically bind to an analyte and/or capture species configured to specifically bind to an analyte, wherein at least one of (i) the detection species and/or capture species and (ii) analyte is a charged species; and wherein the high electric fields are configured such that the electric field can be used to unbind the analyte and the detection species and/or capture species. That is, the analyte is detached from either a capture species, a detection species or both (where both are present) using the structures. In some examples, both may be a charged species. These examples can be used to determine the binding relationship between the analyte and the other species, which can provide further information on the nature of the analyte.

In one example, the system is for determining the property of an analyte, the system comprises the sensor according to any of the examples set out herein; wherein the analyte is a charged analyte; and wherein the sensing assembly is configured to detect the charged analyte after the controller generates an electric field.

In examples, the system may further comprise a controller configured to: determine a property based on the measurement signal. In one example, the system may further comprise a signal processing unit configured to process measurement signals received from the sensor assembly; and the property determination unit may receive processed signals from the signal processing unit. The property determination unit may, in certain examples, be configured to determine the property based on the absolute change in measurement signal and/or the rate of change of the signals. The controller, property determination unit and/or signal processing unit may each (individually or combined) be a processor or controller. The controller may incorporate the property determination unit and/or the signal processing unit or may be in addition to these. The controller, signal processing unit, and the property determination unit may be implemented in any suitable manner, with software and/or hardware, to perform the various functions required. One or all of the units may, for example, employ one or more microprocessors programmed using software (for example, microcode) to perform the required functions. Examples of processor components that may be employed in various examples include, but are not limited to, conventional microprocessors, application specific integrated circuits (ASICs), and field-programmable gate arrays (FPGAs). In various implementations, the controller, the signal processing unit, and/or property determination unit may be associated with one or more non-transitory storage media such as volatile and non-volatile computer memory such as RAM, PROM, EPROM, and EEPROM. The non-transitory storage media may be encoded with one or more programs that, when executed on one or more processors and/or controllers, perform the required functions. Various storage media may be fixed within a processor or controller or may be transportable, such that the one or more programs stored thereon can be loaded into the signal processing unit, property determination unit and/or controller. The system may comprise each of these components, where present, in a single device or at a single location, or the controller and, where present, other components may each individually or as a whole be distributed across a network, such as the internet. The system can also contain antenna, such as RFID structures, such that the sensor outputs can be wirelessly transmitted and/or inputs can be wirelessly transmitted.

In one aspect, a computer program comprising computer program code which is configured, when said computer program is run on one or more physical computing devices, to cause said one or more physical computing devices to implement the methods disclosed herein.

In one aspect, one or more non-transitory computer readable media having a computer program stored thereon, the computer program comprising computer program code which is configured, when said computer program is run on one or more physical computing devices, to cause said one or more physical computing devices to implement the method disclosed herein.

### Specific Examples

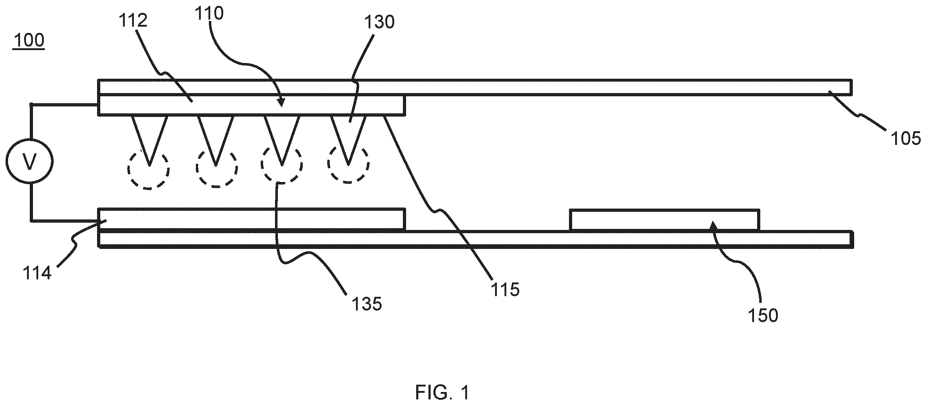

Fig. 1 depicts a schematic cross sectional side view of a sensor 100 for determining a property of a sample containing a charged species. The sensor 100 comprises a fluid channel 105 in which is provided and electric field generator 110 and a sensing assembly 150 downstream of the electric field generator 110 in the fluid channel.

The electric field generator 110 comprises a first field generation element 112 on the upper surface of the fluid channel 105 and a second field generation element 114 spaced apart from the first field generation element 112 and located on the opposite side of the fluid channel 105 to define a region therebetween in which fluid located within the fluid channel 105 can pass. In this example, the first field generation element 112 and the second field generation element 114 are both metal electrodes. These are connected via a trace and can be actuated by applying a voltage to form an electric field between the first and second field generation elements 112, 114, where the electric field forms in the region between the first and second field generation elements 112, 114.

The electric field generator 110 also comprises a plurality of conical structures 130 provided on a first surface 115 defined by lower surface of the first field generation element 112. In this example, these are metallic elements extending from the first surface 115 with the base of the conical shape connected to the first surface 115 and the tip of the conical shape extending into the region, and therefore the centre of the fluid channel 105. The shape, size and material of the plurality of conical structures 130 is such that these concentrate the electric field applied to the region by the first and second field generation elements 112, 114. In particular, these concentrate the electric field at their tip to form corresponding high electric field regions 135 within the region. Accordingly, the electric field generator 110 is configured to generate an electric field between the first field generation element 112 and second field generation element 114 in the region so as to form high electric field region 135 at the tip of the structures 130 such that charged species in a sample received in the fluid channel 105, and hence the region, will interact with the high electric field regions.

The sensing assembly 150 is configured to provide a measurement signal indicative of a property of analyte within or adjacent the region. The measurement signal can be obtained by addressing a part of the sensing assembly 150 and processing the resulting signal to determine the property of the analyte.

Accordingly, in use, a sample can be provided to the fluid channel 105 at or upstream of the electric field generator 110, at which point the electric field generator 110 can be actuated so as to generate an electric field within the fluid channel 105. When this electric field is applied, the structures 130 concentrate the electric field at their tip portions to generate high electric field regions 135, where the electric field is stronger than in the other parts of the fluid channel and region. In one exemplary example, the sample comprises an analyte which is a charged species. Accordingly, as this passes through the region, the analyte is influenced by the high electric field regions, whereas more weekly charged species or species carrying no charge will not be influenced by the high electric field regions. This can be used to influence the sample. For example, analyte could be retained on the tip of the structures 130 while the remainder of this sample is washed through the fluid channel 105. This can concentrate the analyte within the fluid channel 105 and/or act as a filter to remove other species within the sample, such that subsequent measurement at the sensing assembly 150 is improved.

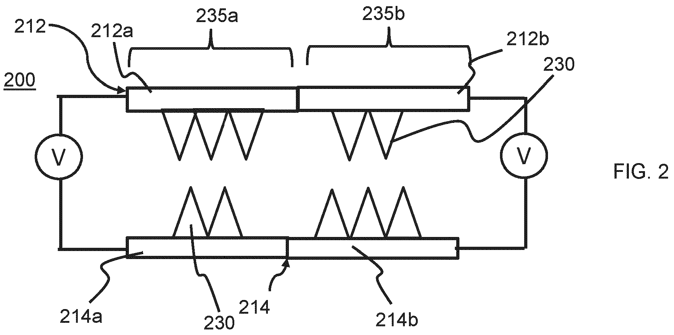

Fig. 2 depicts a schematic cross sectional side view of a device 200 for manipulating a charged species in a sample. The device 200 comprises an electric field generator 210 comprising a first field generation element 212 and a second field generation element 214 spaced apart from the first field generation element 212 and located opposite the first field generation element 212 to define a channel 205 therebetween. In this example, the first field generation element 212 is formed of a first sub-element 212a and a second sub-element 212b and the second field generation element 214 is formed of third sub-element 214a and a fourth sub-element 214b. The first sub-element 212a is opposite to and electrically connected to the third sub-element 214a so that application of a voltage to these results in an electric field therebetween. The second sub-element 212b is opposite to and electrically connected to the fourth sub-element 214b so that application of a voltage to these results in an electric field therebetween. In this example, each of the first to fourth sub-elements 212a, 212b, 214a, 214b is a metal plate electrode. The first and second sub-elements 212a, 212b are electrically insulated from one another and the third and fourth sub-elements 214a, 214b are electrically insulated from one another.

The electric field generator 210 also comprises a plurality of structures 230 provided on both the first field generation element 212 and the second field generation element 214 within the channel 205. In this example, these are metallic elements extending from each of the first field generation element 212 and the second field generation element 214. The tip of the shape of each of the structures 230 extends into the channel 205. The shape, size and material of the plurality of conical structures 230 is such that these concentrate the electric field applied to the region by the first and second field generation elements 212, 214 and, in particular the first to fourth sub-elements 212a, 212b, 214a, 214b. In particular, these concentrate the electric field at their tip to form corresponding high electric field regions 135 within the region.

In this example, the device 200 comprises a first manipulation region 235a and a second manipulation region 235b. The first manipulation region 235a is defined by a first structure set made up of the structures 230 provided on the first sub-element 212a and third sub-element 214a, which can be selectively actuated to provide an electric field in the first manipulation region 235a. This creates high electric fields on the structures 230 of the first manipulation region 235a and can be used to manipulate charged species within this first manipulation region 235a of the channel. The second manipulation region 235b is defined by a second structure set made up of the structures 230 provided on the second sub-element 212b and fourth sub-element 214b, which can be selectively actuated to provide an electric field in the second manipulation region 235b. This creates high electric fields on the structures 230 of the second manipulation region 235b and can be used to manipulate charged species within this second manipulation region 235b of the channel.

Accordingly, in use, a sample can be provided to the channel 205. The electric field generator 210 can be actuated so as to generate an electric field within one of the first or second manipulation regions 235a, 235b within the channel 205. When this electric field is applied, either of the first set of structures 230 or second set of structures 230 concentrate the electric field at their tip portions to generate high electric field regions, where the electric field is stronger than in the other parts of the channel 205. In exemplary examples, the first and second manipulation regions 235a, 235b can be sequentially actuated to move fluid along the channel 205 or back and forth within the channel 205. In some examples, the first and second manipulation regions 235a, 235b may be simultaneously actuated but have different strength electric fields applied thereto so that the high electric field regions in turn are different and may be used to interact with different charged species in different ways. For example, these may be used to retain charged species of different charges.

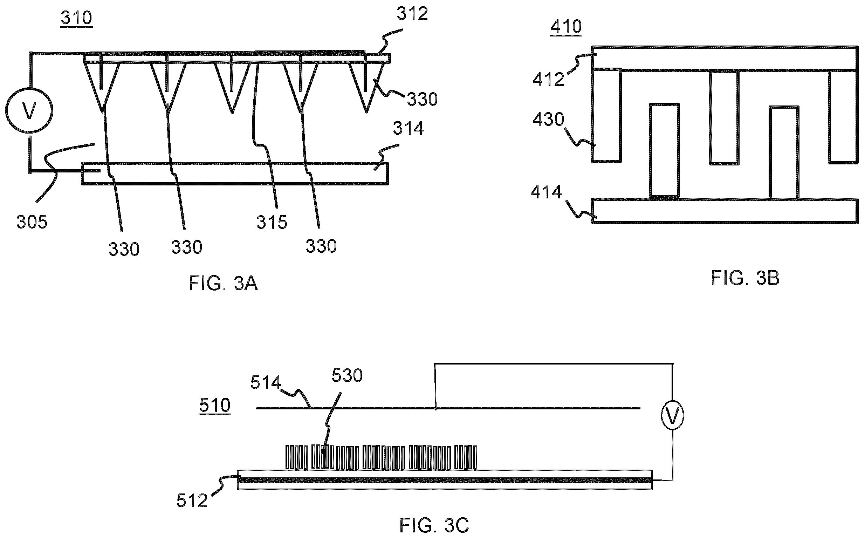

Figs. 3A to 3E provide schematic cross-sectional side views of alternative electric field generators 310, 410, 510, 610, 710 which can be used in the sensors and devices according to examples.

Fig. 3A depicts an electric field generator 310 with a similar structure to that of Fig. 1 in that it comprises a first field generation element 312 and a second field generation element 314 spaced apart from and opposite to the first field generation element 312 to define a fluid channel 305 therebetween. The electric field generator 310 also comprises a plurality of conical structures 330 provided on a first surface 315 defined by lower surface of the first field generation element 312.

However, this electric field generator 310 differs from that of claim 1 in that each of the structures 330 is individually electrically connected to the second field generation element 314 such that each can form its own sub-element and hence together with the second field generation unit 314 each forms a manipulation region. This electric field generator 310 accordingly has a plurality of sub-elements formed of each structure 330. This can also be in addition to the ability to actuate all (or a combination) of the structures 330 together using the first and second field generation elements 312, 314. This allows for each sub-element to be sequentially addressed along the length of the region so that charged species can be moved along the length of the region.

In a further modification, the second field generation element 314 may be formed of sub-elements (not shown) which correspond to the sub-elements of the first field generation element 312 to form a manipulation regions. In this way, each manipulation region can be actuated simultaneously. For example, this can be used with different field strengths to create gradients along the length of the region.

Fig. 3B shows an electric field generator 410 having opposing first and second field generation elements 412, 414 in a similar structure to the above example of Fig. 1, with first and second field generation elements 412, 414 defining a channel therebetween and being electrically connected so that a field can be formed in the channel. In this example, however, there are opposing and offset structures 430 forming rows of interlocking teeth on each of the first and second field generation elements 412, 414. This creates a tortuous path through the channel which can be characterised by higher fields on the structures 430. This can be used to filter and retain charged species, for example.

Fig. 3C depicts an electric field generator 510 with a similar structure to that of Fig. 1 in that it comprises a first field generation element 512 and a second field generation element 514 spaced apart from and opposite to the first field generation element 512 and with a plurality of structures 530 formed on the region-facing surface of the first field generation element 512. This electric field generator 510 differs in that the second field generation element 514 is a wire which extends over the length of the first field generation element 512. Additionally, in this example the structures 530 are a series of elongated wires extending up from the region-facing surface of the first field generation element 512. These can be, for example, gold nanowires.

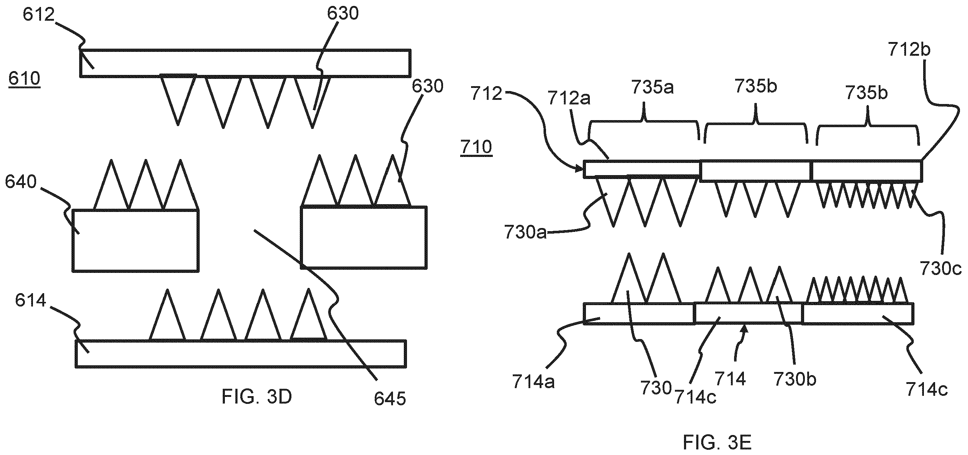

Fig. 3D depicts a further electric field generator 610 and a part of a sensing assembly. The electric field generator 610 has opposing first and second field generation elements 612, 614 in a similar structure to the above example of Fig. 1, with first and second field generation elements 612, 614 defining a region therebetween and being electrically connected so that an electric field can be formed in the channel. In this example, there are opposing structures 430 formed on each of the inwardly facing faces of the first and second field generation elements 612, 614 (first and second surfaces, respectively). These structures 630 are used to concentrate the electric field in a similar manner to Fig. 1.

In addition to these, a sensing layer 640 forming part of a sensing assembly is provided between the opposing first and second field generation elements 612, 614. This sensing layer 640 comprises a through hole 645 which is responsive to the presence of an analyte therein. Further structures 630 are provided around the opening of the through hole 645 and also serve to create high electric field regions around their tip. The presence of these structures 630, and also those on the first and second surfaces, helps to manipulate analyte relative to the through hole 645 thereby allowing for modulation of the measurement signal and further information about an analyte to be determined and more quickly.

Fig. 3E depicts a schematic cross sectional side view of an electric field generator 710 having a similar structure to that of Fig. 2. The electric field generator 710 comprises a first field generation element 712 and a second field generation element 714 spaced apart from the first field generation element 712 and located opposite the first field generation element 712 to define a region therebetween. In this example, the first field generation element 712 is formed of a first sub-element 712a, a second sub-element 712b and a third sub-element 712c. The second field generation element 714 is formed of fourth sub-element 714a, a fifth sub-element 714b and a sixth sub-element 714c. The first sub-element 712a is opposite to and electrically connected to the fourth sub-element 714a so that application of a voltage to these results in an electric field therebetween. The second sub-element 712b is opposite to and electrically connected to the fifth sub-element 714b so that application of a voltage to these results in an electric field therebetween. The third sub-element 712c is opposite to and electrically connected to the sixth sub-element 714x so that application of a voltage to these results in an electric field therebetween. In this example, the sub-elements forming each of the first and second field generation elements 712, 714 are electrically insulated from one another.

In this example, the electric field generator 710 also comprises a plurality of structures 730a-c provided on the first field generation element 712 and second field generation element 714. These together with the first to sixth sub-elements 712a-c, 714a-c define three manipulation regions: the first manipulation region 735a comprises a first structure set made up of the structures 730a provided on the first sub-element 712a and fourth sub-element 714a, which can be selectively actuated to provide an electric field in the first manipulation region 735a; the second manipulation region 735b is defined by a second structure set made up of the structures 730 provided on the second sub-element 712b and fifth sub-element 714b; and the third manipulation region 735c is defined by a third structure set made up of the structures 730c provided on the third sub-element 712c and sixth sub-element 714c.

In a further difference to the device 200 of Fig. 2, the structures 730a-c of the first to third manipulation regions 735a-c are different to one another. In particular, the structures 730a of the first manipulation region 735a are the largest (in surface area and height of each structure); the structures 730c of the smallest manipulation region 735c are the smallest; and the structures 730b of the second manipulation region 735b are a size in between the size of the other structures 730a, 730c. There are also more structures in the third manipulation region 735c than the first and second manipulation regions 735a, 735b. This can be used to create a gradient across the first and second field generation elements 712, 714 which can be used to manipulate charged species, for example to drive them across the surfaces and through the region.

In an alternative example of the electric field generator 710, the first and second field generation elements 712, 714 may only be comprised of a single electric field generation element (each). That is, there need not be sub-elements provided for each grouping of structures 730a-730b. Instead, the size of the structures 730a-730c may vary across a single-actuatable surface of a field generation element. This can also be used to create a gradient.

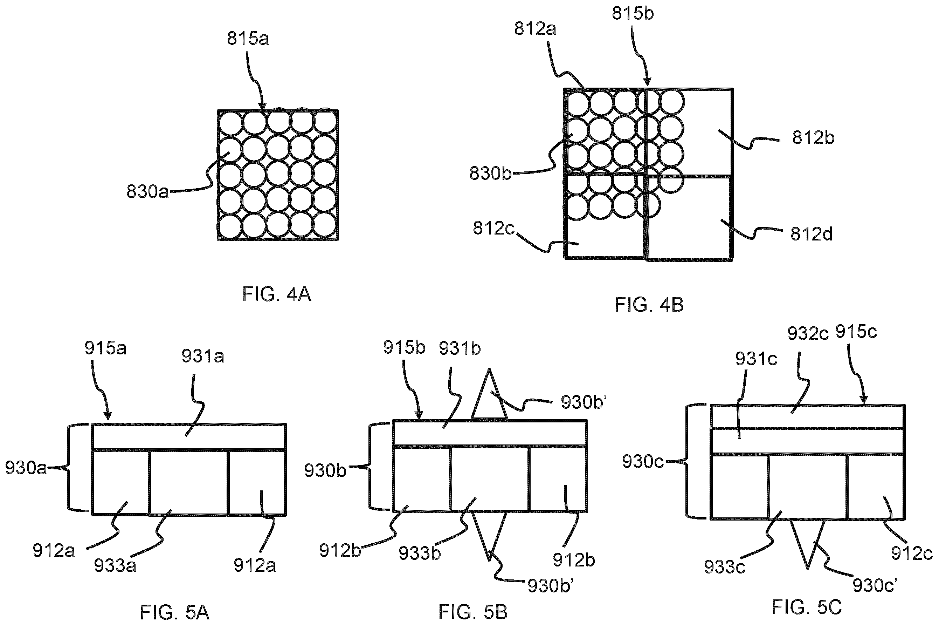

Figs. 4A and 4B depict other exemplary ways of providing structures on surfaces. Fig. 4A and 4B depict first surfaces 815a, 815b in which structures 830a, 830b are formed on using self-assembled monolayers (SAMs) formed from beads, such as metallic or dielectric beads (e.g. gold, polystyrene and/or silica beads). In both instances, the beads can form into lattices with the electric field concentrated in regions around the beads (such as between the beads).

In Fig. 4A, there is provided an electrode 812 on which the structures 830a are formed. A voltage can be applied to this (with a corresponding second electrode - not shown) to generate an electric field which is concentrated by the structures 830a. The example of Fig. 4B differs in that the electrode 812 forming the first surface 815b is comprised of a plurality of sub-elements 812a-812d. Each of these sub-elements 812a-812d can individually have a voltage applied thereto. In this way, each of sub-elements 812a-812d could be selectively actuated to provide manipulation in a different region and improve efficiency.

Figs. 5A to 5C depict other exemplary ways of providing structures for concentrating and shaping electric fields. Each of Figs. 5A to 5C depicts a part of a first electrode element 912a-912c in which at least one through hole 933a-933c is formed. In each of these examples, the through hole 933a-933c is filled with a different material ("first material") to the respective first electrode element 912a-912c in which it is formed. The first material has a first dielectric constant ε. In each of the examples, the first electrode element 912a-912c is covered by a layer 931a-931c, which layer covers the respective through hole(s) 933a-933c. The layer 931a-931c is formed from a second material, which is different from the first material, and has a second dielectric constant ε₁. In this example dielectric constant ε₁ > ε. Permittivity differences can impact the electric field and be used to focus and/or steer the electric field. Together the through holes 933a-933c, first material and second materials (layer) define a structure 930a-930c. The part of the electric field generators depicted in Figs. 5B and 5C also comprise further structures. In the case of Fig. 5B, further conical or pyramidal structures 930b' are provided on the upper and lower surfaces of the first electrode element 915b above and below the through hole 933b to further shape and concentrate the electric fields. In Fig. 5B, a further conical or pyramidal structure 930c' is provided on the lower surface of the first electrode element 915c below the through hole 933b to further shape and concentrate the electric fields. Further, a further material is provided on top of the layer 931c forming the second material. This can be a second layer formed of a third material with a third dielectric constant, for example, or can in examples be a channel or pore filled with an electrolyte.

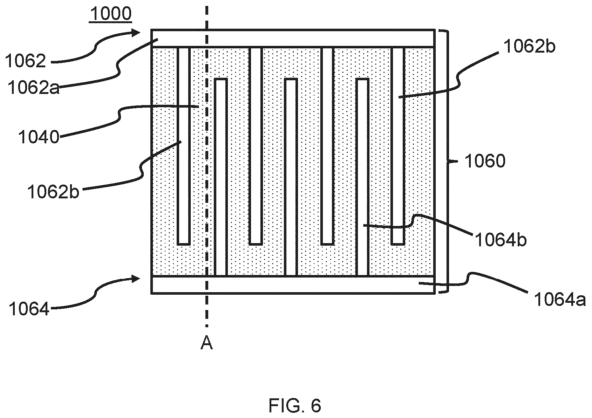

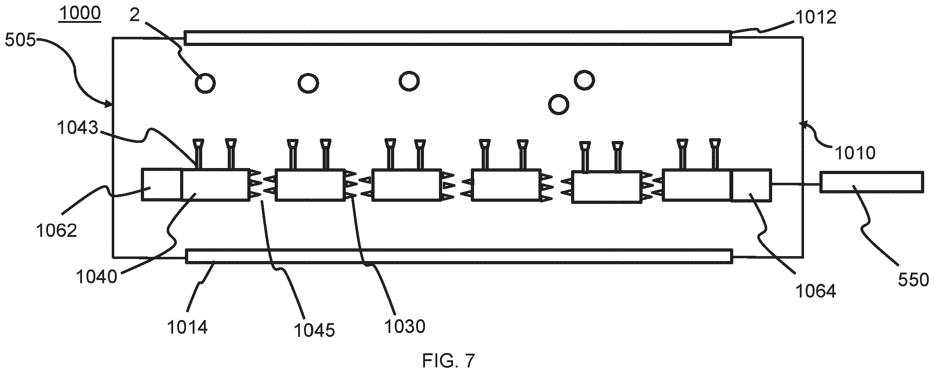

Figs. 6 and 7 schematically depict a further example of a sensor 1000 for determining a property of an analyte. Fig. 6 provides a schematic plan view of a part of the sensor 1000, including the sensing assembly 1050, and Fig. 7 provides a schematic cross-sectional view of the sensor 1000 with sensing assembly 1050 taken along line A shown in Fig. 6.

The sensing assembly 1050 comprises a sensing element which comprises a sensing layer 1040 and a measurement IDE 1060 formed from a first measurement electrode 1062 and a second measurement electrode 1064 arranged in an interdigitated configuration. The first measurement electrode 1062 comprises a first connector 1062a which extends along one side of the sensing layer 1040 and from which plural first extension portions 1062b extend perpendicularly. The second measurement electrode 1064 comprises a second connector 1064a which extends along the opposite side of the sensing layer 1040 to the first connector 1062a and plural second extension portions 1064b extend perpendicularly from the second connector 1064a towards the first connector 1062a. The second extension portions 1064b are received between the first extension portions 1062b in an interdigitated comb configuration. The sensing layer 1040 in this example is functionalised on a part of the upper surface with capture species 1043 configured to specifically bind with the analyte 2 provided on the upper surface to form a sensing surface. The measurement IDE 1060 surrounds the sensing surface of the sensing layer 1040 and interrogates the sensing layer 1040 in this region to provide a measurement signal indicative of a property of the analyte 2.

The sensor 1000 also comprises an electric field generator 1010 (see Fig. 7, in particular) which is configured to apply a field to the sensing surface so as to apply a force to any sample adjacent the sensing surface, as will be set out in more detail below. The electric field generator 1010 in this example applies an electric field to the sample and comprises a first electrode 1012 provided above the sensing layer 1040 and a second electrode 1014 electrically connected to the first electrode 1012 and located below the sensing layer 1040. The first electrode 1012 and second electrode 1014 are configured to apply an electric field through the sample and about the sensing layer 1040 - i.e. where the interaction between capture species 1043 and analyte 2 will occur.

The sensor assembly 1050 in this example comprises a plurality of through holes 1045 extending from an upper surface to a lower surface and across the sensing layer 1040 (as can be seen in Fig. 7). The sensing layer 1040 is suspended in a chamber such that fluid resides on both sides of the sensing layer 1040 and with the through holes 1045 providing for passage of fluid from the region above the sensing layer 1040 to below the sensing layer 1040. The capture species 1043 are provided on the upper surface adjacent the through holes 1045. The measurement IDE 1060 is therefore provided with the through holes 1045 of the sensing layer 1040 between the interdigitated portions of the first measurement electrode 1062 and second measurement electrode 1064. Moreover, in this example, each of the through holes 1045 is provided with a plurality of structures 1030 located on its inner surface(s). These structures 1030 can be in accordance with any of the structures set out herein, including conical structures, and are configured to concentrate the electric field generated by the electric field generator using the first electrode 1012 and second electrode 1014. This can also amply the signal in the pores using the measurement IDE 1060, where the first and second measurement electrodes 1062, 1064 form first and second field generation elements and the structures 1030 concentrate this field as well.

The use of the sensing layer 1040 with through holes 1045, or pores, provides a particularly useful tool for determining a property of an analyte. The configuration of the sensing layer 1040 and the capture species 1044 is such that analyte that is specifically bound to the capture species is able to modify an impedimetric property (e.g. relative permittivity or dielectric constant (both referred to herein as "permittivity")) of the sensing layer. As such, the relative position of the analyte within or around the through hole 1045 can have a significant effect on the measurement signal and can provide detailed information on the particular analyte, in addition to the information provided by the selective binding of the analyte to the capture species. Prior to interaction of the sensing layer with the sample, the impedimetric property of the sensing layer is determined by the sensing layer and any fluid within the through holes. However, once a species is received within through hole(s), the species will cause a change in this impedimetric property. Depending on the analyte property and its modulation of the signal the property to be measured may be due to double layer change or interruption, sensing layer thickness increase, faradaic or non-faradaic processes, transfer of charge, charge storage, or charge induction. For example, when an analyte is bound to the capture species and can interact with the through hole, such as enter and traverse through the through hole, the analyte (and/or a tag or detection species, which may enable the interaction with the through holes) will contribute to the impedimetric property of the sensing layer. It will be appreciated that this could be present in any of the examples, and that it is not limited to a particular modification unit or measurement electrode structure.

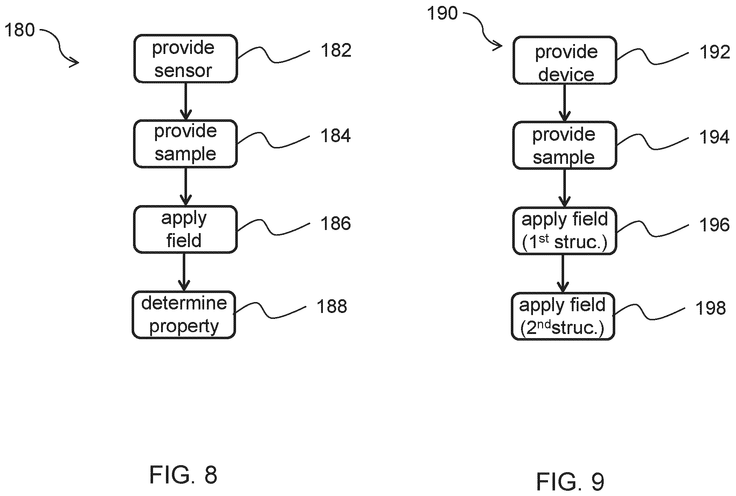

Fig. 8 depicts a method 180 of determining a property of a sample, the method 180 comprising:
providing a sensor 182 comprising:
   a first electrode element; a second electrode element spaced apart from the first electrode element to define a region therebetween; and
   a plurality of structures provided on a first surface within the region, wherein each of the plurality of structures is configured to concentrate an applied electric field to form corresponding high electric field regions within the region; and
   a sensing assembly configured to provide a measurement signal indicative of a property of sample within or adjacent the region,
providing a sample to the region 184,
applying an electric field 186 to at least one of the plurality of structures to create a high electric field region within the region so as to manipulate charged species within the sample; and
determining a property of the sample 188 based on the measurement signal after manipulation of the charged species.

Fig. 9 depicts a method 190 of manipulating a charged species in a sample, the method 190 comprising:
providing a device 192 comprising:
   an electric field generator comprising a first electrode element; a second electrode element spaced apart from the first electrode element to define a region therebetween; and a plurality of structures provided within the region, wherein each of the plurality of structures is configured to concentrate an applied electric field to form corresponding high electric field regions within the region;
providing a sample to the region 194;
selectively applying an electric field 196 to a first structure set comprising at least one of the plurality of the structures to create a first manipulation region to thereby manipulate a charged species; and
selectively applying an electric 198 field to a second structure set comprising at least one of the plurality of the structures to create a second manipulation region to thereby manipulate a charged species,
wherein the first structure set and second structure set are different.

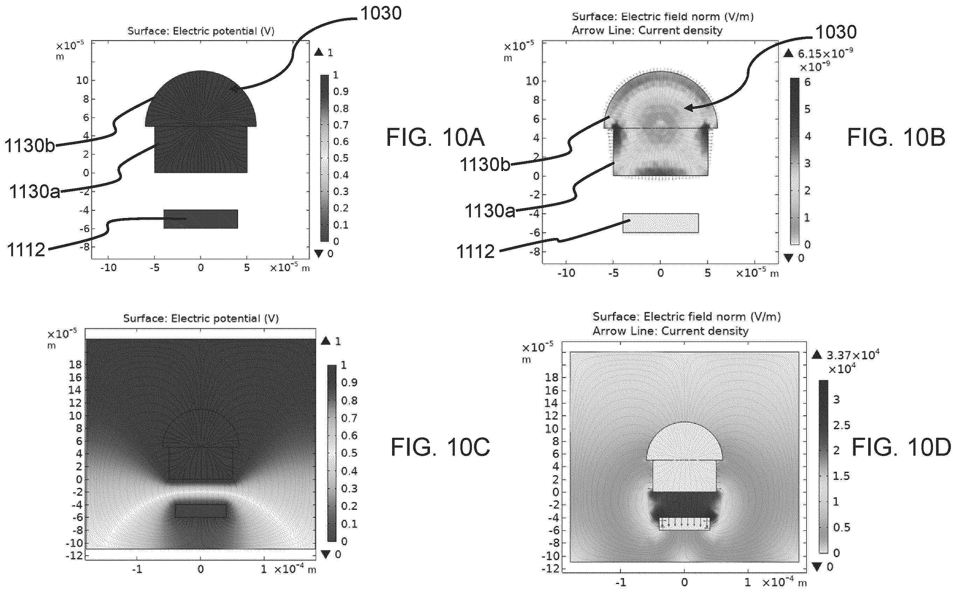

As set out above, the structures can be provided with various profiles to concentrate the electrical field. In some embodiments, the structures are spaced apart from the first and/or second electrode elements. Figs. 10A to 10D depict an exemplary structure 1130, where the structure 1130 is provided on or as a second electrode element and comprises a mushroom-type profile. That is, the structure 1130 has a profile with a base portion 1130a of a first width and a semi-circular tip 1130b provided on the base portion, where the tip 1130b extends beyond the width of the base portion 1130a. Figs. 10A to 10B show the arrangement where the voltage is applied between the first electrode element 1112 and the structure 1130. Fig. 10A shows the electric potential (V) being higher on the structure 1130 than on the first electrode element 1112 and Fig. 10B shows the electric field norm (V/m) and current density (arrow line) demonstrating the presence of higher concentrations in particular regions, such as the base of the mushroom shape and the join of the base portion and the tip portion. Figs. 10C and 10D depict the arrangement there is an electrolyte between the structure 1130 and the first electrode element 1112 and depicts the concentration of the electric filed therebetween. Fig. 10C shows the electric potential (V) being higher on the structure 1130 and Fig. 10D shows the electric field norm (V/m) and current density (arrow line) demonstrating the presence of higher concentrations in particular regions, such as the base of the mushroom shape and between the surfaces. These demonstrate how these shapes can be used to manipulate electric fields within the region.

It should be understood that the detailed description and specific examples, while indicating exemplary examples of the apparatus, systems and methods, are intended for purposes of illustration only and are not intended to limit the scope. These and other features, aspects, and advantages of the apparatus, systems and methods of the present disclosure can be better understood from the description, appended claims or aspects, and accompanying drawings. It should be understood that the Figures are merely schematic and are not drawn to scale. It should also be understood that the same reference numerals are used throughout the figures to indicate the same or similar parts.

Other variations to the disclosed examples can be understood and effected by those skilled in the art in practicing the disclosure, from a study of the drawings, the disclosure, and the appended aspects or claims. In the aspects or claims, the word "comprising" does not exclude other elements or steps, and the indefinite article "a" or "an" does not exclude a plurality. The mere fact that certain measures are recited in mutually different dependent aspects or claims does not indicate that a combination of these measures cannot be used to advantage. Any reference signs in the claims should not be construed as limiting the scope.

Examples of the disclosure will now be set out, in which:
Example 1: A sensor for determining a property of a sample, the sensor comprising:
   an electric field generator for forming an electric field, comprising:
      a first electrode element,
      a second electrode element spaced apart from the first electrode element to define a region therebetween, wherein the electric field generator is configured to generate an electric field between the first electrode element and the second electrode element in the region; and
   a sensing assembly configured to provide a measurement signal indicative of a property of a sample provided within or adjacent the region,
   wherein the electric field generator further comprises a plurality of structures provided within the region and a first surface comprising at least one structure of the plurality structures; and
   wherein each of the plurality of structures is configured to concentrate an electric field applied to the region by the electric field generator to form corresponding high electric field regions such that charged species in a sample provided to the region can interact with the high electric field regions.
Example 2: The sensor of example 1, wherein each structure of the plurality of structures has a height of from 10 nm to 10 µm.
Example 3: The sensor of example 1 or example 2, wherein the first electrode element and the second electrode element define a fluid inlet to the region and an opposing fluid outlet from the region, and the plurality of structures are arranged along the length of the region between the fluid inlet and fluid outlet.
Example 4: The sensor of example 3, wherein the plurality of structures are varied in configuration along the length of the region so as to alter the strength of an applied electric field in different parts of the region.
Example 5: The sensor of example 4, wherein the configuration of the plurality of structures comprises the number of structures per unit area and/or the spatial configuration of the structures along the length of the region and/or wherein the plurality of structures varying in configuration by at least one of the height, width, total volume, and/or shape of each structure.
Example 6: The sensor of any preceding example, wherein each of the plurality of structures comprises a protrusion extending from the first surface, each protrusion comprising a base portion connected to the first surface and a tip portion connected to the base portion; and wherein the tip portion has a width which is different to the base portion.
Example 7: The sensor of any preceding example, wherein at least one structure of the plurality of structures is provided on a second surface, wherein the second surface is provided opposite the first surface in the region.
Example 8: The sensor of any preceding example, wherein the sensor comprises a first manipulation region defined by a first structure set comprising at least one of the plurality of structures; and a second manipulation region defined by a second structure set comprising at least one of the plurality of structures; and
   wherein the electric field generator is configured to selectively apply an electric field to each of the first manipulation region and the second manipulation region.
Example 9: The sensor of any preceding example, wherein the sensing assembly comprises a sensing element, the sensing element comprising an upper surface and a lower surface and a through hole, the through hole extending from the upper surface to the lower surface; and wherein sensing element is configured to provide the measurement signal, the measurement signal being indicative of the interaction of a sample with the sensing element.
Example 10: The sensor of example 9, wherein the sensing element comprises the first surface.
Example 11: A device for manipulating a charged species in a sample, the device comprising:
   an electric field generator for forming an electric field, comprising:
      a first electrode element; and
      a second electrode element spaced apart from the first electrode element to define a region therebetween, wherein the electric field generator is configured to generate an electric field between the first electrode element and the second electrode element in the region,
   wherein the electric field generator further comprises a plurality of structures provided within the region;
   wherein each of the plurality of structures is configured to concentrate an electric field applied to the region by the electric field generator to form corresponding high electric field regions such that charged species in a sample provided to the region can interact with the high electric field regions.
   wherein the device comprises a first manipulation region defined by a first structure set comprising at least one of the plurality of structures; and a second manipulation region defined by a second structure set comprising at least one of the plurality of structures; and
   wherein the electric field generator is configured to selectively apply an electric field to each of the first manipulation region and the second manipulation region.
Example 12: The device of example 11, wherein at least one structure of the plurality of structures is provided on a first surface provided within the region and at least one structure of the plurality of structures is provided on a second surface provided within the region, wherein the second surface is opposite the first surface.
Example 13: The device of example 12, wherein the at least one structure of the first manipulation region is provided on the first surface and wherein the at least one structure of the second manipulation region is provided on the second surface.
Example 14: The device of any of examples 11 to 13, wherein the first manipulation region and the second manipulation region are spaced apart so as to form discrete manipulation regions.
Example 15: A system for determining a property of a sample, the system comprising:
   a sensor according to any of examples 1 to 10 and/or the device according to any of examples 11 to 14; and
   a controller configured to control the generation of an electric field by the electric field generator.
Example 16: The system of example 15, wherein the system is for determining the property of an analyte,
   wherein the system further comprises a detection species configured to specifically bind to an analyte and/or a capture species configured to specifically bind to an analyte, wherein at least one of the detection species and/or capture species and analyte is a charged species; and
   wherein the electric field generator and plurality of structures are configured such that the high electric field regions can be used to cause an analyte to debind from the detection species and/or capture species.
Example 17: The system of example 15 or example 16, wherein the system is for determining the property of an analyte and comprises the sensor according to any of examples 1 to 10;
   wherein the analyte is a charged analyte; and
   wherein the system is configured such that sensing assembly is configured to detect the charged analyte after the controller generates an electric field.
Example 18: A method of determining a property of a sample, the method comprising:
   providing a sensor comprising:
      an electric field generator for forming an electric field, comprising:
         a first electrode element,
         a second electrode element spaced apart from the first electrode element to define a region therebetween;
         a plurality of structures provided within the region;
         a first surface comprising at least one structure of the plurality structures,
         wherein each of the plurality of structures is configured to concentrate an applied electric field to form corresponding high electric field regions within the region; and
      a sensing assembly configured to provide a measurement signal indicative of a property of sample within or adjacent the region,
   providing a sample to the region,
   applying an electric field using the electric field generator to at least one of the plurality of structures to create a high electric field region within the region so as to manipulate charged species within the sample; and
   determining a property of the sample based on the measurement signal after manipulation of the charged species.
Example 19: The method of example 18,
   wherein the device comprises a first manipulation region defined by a first structure set comprising at least one of the plurality of structures; and a second manipulation region defined by a second structure set comprising at least one of the plurality of structures; and
   wherein applying an electric field comprises:
   selectively applying an electric field to the first structure set; and
   selectively applying an electric field to a second structure set.
Example 20: The method of example 19, comprising:
   moving charged species within the region by sequentially applying an electric field to the first structure set and to the second structure set.
Example 21: The method of example 19 or example 20, wherein at least one structure of the plurality of structures is provided on a second surface provided within the region and opposing the first surface; and
   wherein applying an electric field comprises selectively applying an electric field to at least one of the plurality of structures on the first surface; and selectively applying an electric field to at least one of the plurality of structures on the second surface.
Example 22: The method of any of examples 18 to 21, wherein the method is for detecting a property of an analyte in the sample and wherein the method further comprises:
   providing a detection species configured to specifically bind to an analyte and/or a capture species configured to specifically bind to an analyte to the region, wherein at least one of the detection species and/or capture species and analyte is a charged species; and
   wherein applying an electric field comprises applying an electric field to form a high electric field regions and causing the detection species and/or capture species to debind from the analyte using the high electric field regions.
Example 23: The method of any of examples 18 to 22, wherein applying an electric field comprises applying an electric field so as to retain charged species within the high electric field regions; and wherein the method further comprises moving sample through the region while retaining the charged species within the high electric field regions.
Example 24: A method of manipulating a charged species in a sample, the method comprising:
   providing an electric field generator for forming an electric field, the electric field generator comprising:
      a first electrode element,
      a second electrode element spaced apart from the first electrode element to define a region therebetween, wherein the electric field generator is configured to generate an electric field between the first electrode element and the second electrode element in the region; and
   a plurality of structures provided within the region, wherein each of the plurality of structures is configured to concentrate an applied electric field to form corresponding high electric field regions within the region; and
   providing a sample to the region;
   selectively applying an electric field to a first structure set comprising at least one structure of the plurality of the structures to create a first manipulation region to thereby manipulate a charged species; and
   selectively applying an electric field to a second structure set comprising at least one structure of the plurality of the structures to create a second manipulation region to thereby manipulate a charged species.
Example 25: The method of example 24, wherein the method comprises moving charged species within the region by sequentially applying an electric field to the first structure set and to the second structure set.
Example 26: The method of example 24 or example 25, wherein at least one of the plurality of structures is provided on a first surface provided within the region and wherein at least one of the plurality of structure is provided on a second surface provided within the region and opposite the first surface; and
   wherein the first structure set comprises at least one structure of the plurality of structures on the first surface and the second structure set comprises at least one structure of the plurality of structures on the second surface.
Example 27: The method of any of examples 24 to 26,
   wherein applying an electric field to at least one of the first structure set or the second structure set comprises applying an electric field to at least one of the first structure set or the second structure set so as to retain charged species within the high electric field region; and
   wherein the method comprises moving sample through the region while retaining the charged species within the high electric field region.

## Claims

1. A sensor for determining a property of a sample, the sensor comprising:
an electric field generator for forming an electric field, comprising:
a first electrode element,
a second electrode element spaced apart from the first electrode element to define a region therebetween, wherein the electric field generator is configured to generate the electric field between the first electrode element and the second electrode element in the region; and
a sensing assembly configured to provide a measurement signal indicative of a property of a sample provided within or adjacent the region,
wherein the electric field generator further comprises a plurality of structures provided within the region and a first surface comprising at least one structure of the plurality of structures; and
wherein each of the plurality of structures is configured to concentrate the electric field applied to the region by the electric field generator to form corresponding high electric field regions such that charged species in the sample provided to the region can interact with the high electric field regions.

2. The sensor of claim 1, wherein each structure of the plurality of structures has a height of from 10 nm to 10 µm.

3. The sensor of claim 1 or claim 2, wherein the first electrode element and the second electrode element define a fluid inlet to the region and an opposing fluid outlet from the region, and the plurality of structures are arranged along a length of the region between the fluid inlet and fluid outlet.

4. The sensor of claim 3, wherein the plurality of structures are varied in configuration along the length of the region so as to alter a strength of an applied electric field in different parts of the region; optionally, wherein the configuration of the plurality of structures comprises a number of structures per unit area and/or a spatial configuration of the plurality of structures along the length of the region and/or wherein the plurality of structures varying in configuration by at least one of a height, width, total volume, and/or shape of each structure.

5. The sensor of any preceding claim, wherein each of the plurality of structures comprises a protrusion extending from the first surface, each protrusion comprising a base portion connected to the first surface and a tip portion connected to the base portion; and wherein the tip portion has a width which is different to the base portion.

6. The sensor of any preceding claim, wherein at least one structure of the plurality of structures is provided on a second surface, wherein the second surface is provided opposite the first surface in the region.

7. The sensor of any preceding claim, wherein the sensor comprises a first manipulation region defined by a first structure set comprising at least one of the plurality of structures; and a second manipulation region defined by a second structure set comprising at least one of the plurality of structures; and
wherein the electric field generator is configured to selectively apply the electric field to each of the first manipulation region and the second manipulation region.

8. A device for manipulating a charged species in a sample, the device comprising:
an electric field generator for forming an electric field, comprising:
a first electrode element; and
a second electrode element spaced apart from the first electrode element to define a region therebetween, wherein the electric field generator is configured to generate the electric field between the first electrode element and the second electrode element in the region,
wherein the electric field generator further comprises a plurality of structures provided within the region;
wherein each of the plurality of structures is configured to concentrate the electric field applied to the region by the electric field generator to form corresponding high electric field regions such that charged species in a sample provided to the region can interact with the high electric field regions.
wherein the device comprises a first manipulation region defined by a first structure set comprising at least one of the plurality of structures; and a second manipulation region defined by a second structure set comprising at least one of the plurality of structures; and
wherein the electric field generator is configured to selectively apply the electric field to each of the first manipulation region and the second manipulation region.

9. The device of claim 8, wherein at least one structure of the plurality of structures is provided on a first surface provided within the region and at least one structure of the plurality of structures is provided on a second surface provided within the region, wherein the second surface is opposite the first surface.

10. The device of claim 9, wherein the at least one structure of the first manipulation region is provided on the first surface and wherein the at least one structure of the second manipulation region is provided on the second surface.

11. A method of determining a property of a sample, the method comprising:
providing a sensor comprising:
an electric field generator for forming an electric field, comprising:
a first electrode element,
a second electrode element spaced apart from the first electrode element to define a region therebetween;
a plurality of structures provided within the region;
a first surface comprising at least one structure of the plurality of structures,
wherein each of the plurality of structures is configured to concentrate the electric field to form corresponding high electric field regions within the region; and
a sensing assembly configured to provide a measurement signal indicative of a property of a sample within or adjacent the region,
providing the sample to the region,
applying the electric field using the electric field generator to at least one of the plurality of structures to create at least one high electric field region within the region so as to manipulate charged species within the sample; and
determining the property of the sample based on the measurement signal after manipulation of the charged species.

12. The method of claim 11, wherein the sensor comprises a first manipulation region defined by a first structure set comprising at least one of the plurality of structures; and a second manipulation region defined by a second structure set comprising at least one of the plurality of structures; and
wherein applying the electric field comprises:
selectively applying the electric field to the first structure set; and
selectively applying the electric field to the second structure set.

13. The method of claim 12, wherein the method is for detecting the property of an analyte in the sample and wherein the method further comprises:
providing a detection species configured to specifically bind to the analyte and/or a capture species configured to specifically bind to the analyte to the region, wherein at least one of the detection species and/or the capture species and the analyte is the charged species; and
wherein applying the electric field comprises applying the electric field to form the at least one high electric field region and causing the detection species and/or capture species to debind from the analyte using the at least one high electric field region.

14. The method of claim 12 or claim 13, wherein applying the electric field comprises applying the electric field so as to retain the charged species within the at least one high electric field region; and wherein the method further comprises moving the sample through the region while retaining the charged species within the at least one high electric field region.

15. A method of manipulating a charged species in a sample, the method comprising:
providing an electric field generator for forming an electric field, the electric field generator comprising:
a first electrode element;
a second electrode element spaced apart from the first electrode element to define a region therebetween, wherein the electric field generator is configured to generate the electric field between the first electrode element and the second electrode element in the region; and
a plurality of structures provided within the region, wherein each of the plurality of structures is configured to concentrate the electric field to form corresponding high electric field regions within the region; and
providing a sample to the region;
selectively applying the electric field to a first structure set comprising at least one first structure of the plurality of the structures to create a first manipulation region to thereby manipulate a charged species; and
selectively applying the electric field to a second structure set comprising at least one second structure of the plurality of the structures to create a second manipulation region to thereby manipulate the charged species.
